(19) **Europäisches Patentamt / European Patent Office / Office européen des brevets**

(11) **EP 3 539 660 B1**

(12) **EUROPÄISCHE PATENTSCHRIFT**

(45) Veröffentlichungstag und Bekanntmachung des Hinweises auf die Patenterteilung:
**01.05.2024   Patentblatt 2024/18**

(21) Anmeldenummer: **18162369.5**

(22) Anmeldetag: **16.03.2018**

(51) Internationale Patentklassifikation (IPC):
**B01L 1/02** *(2006.01)*      **B01L 7/00** *(2006.01)*
**C12M 1/00** *(2006.01)*      **C12M 3/06** *(2006.01)*
**G01K 7/42** *(2006.01)*

(52) Gemeinsame Patentklassifikation (CPC):
**C12M 41/14; B01L 1/025; C12M 27/16;** B01L 7/00;
B01L 2200/147

(54) **LABORTEMPERIERVORRICHTUNG UND VERFAHREN**

LABORATORY TEMPERATURE CONTROL DEVICE AND METHOD

DISPOSITIF DE LA MISE EN TEMPÉRATURE D'UN LABORATOIRE ET PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.09.2019   Patentblatt 2019/38**

(73) Patentinhaber: **Eppendorf SE**
**22339 Hamburg (DE)**

(72) Erfinder:
• **Stöhrer, Frederic**
**22339 Hamburg (DE)**

• **Schuenemann, Patrick**
**22339 Hamburg (DE)**
• **Timmann, Lutz**
**22339 Hamburg (DE)**
• **Maltzen, Hauke**
**22339 Hamburg (DE)**

(74) Vertreter: **Wallinger Ricker Schlotter Tostmann Patent- und Rechtsanwälte mbB**
**Zweibrückenstraße 5-7**
**80331 München (DE)**

(56) Entgegenhaltungen:
GB-A- 2 438 683      JP-A- 2001 321 681
US-A- 6 063 619      US-B1- 6 518 059

**Beschreibung**

Die Erfindung betrifft eine Labortemperiervorrichtung zum Temperieren von Laborproben bei einer Zieltemperatur. Sie betrifft insbesondere einen Inkubator für das Wachstum von Zellkulturen. Zudem betrifft die Erfindung ein Verfahren zum Einstellen einer Zieltemperatur in dieser Labortemperiervorrichtung. Die Erfindung betrifft auch ein Verfahren zur Schätzung der Temperatur im Innenraum der Kammer dieser Labortemperiervorrichtung.

Labortemperiervorrichtungen werden benötigt, um Laborproben in einer abgeschirmten Umgebung bei einer bestimmten Zieltemperatur zu halten, insbesondere aufzubewahren. Mit Inkubatoren werden in biologischen und medizinischen Laboratorien Zellen in Zellkultur unter kontrollierten Umgebungsbedingungen gehalten, um so das Wachstum lebender Zellen *in vitro* zu ermöglichen. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen müssen in $CO_2$-Inkubatoren kultiviert werden. Die Atmosphäre wird durch Luft mit einem bestimmten $CO_2$- und $O_2$-Gehalt und einer bestimmten Luftfeuchtigkeit gebildet, eine geeignete Temperatur ist oftmals 37 °C. Solche Labortemperiervorrichtungen weisen ein die Inkubatorkammer umgebendes Gehäuse auf, beispielsweise ein Außengehäuse, mit einer Gehäuseöffnung, durch die der Benutzer die Proben im Gehäuseinneren, insbesondere in der Inkubatorkammer, lagert und wieder entnimmt.

Eine Kernanforderung an Labortemperiervorrichtungen ist die genaue Einhaltung bzw. Einstellung der vom Benutzer gewünschten Zieltemperatur im Inneren der Kammer der Labortemperiervorrichtung. Inkubatoren weisen üblicher Weise einen Regelkreis auf, dem als Messglied ein Temperatursensor im Inneren der Kammer oder in Verbindung mit dem Inneren der Kammer zugeordnet ist und dem als Stellglied die Temperiereinrichtungen des Inkubators zugeordnet ist. Entscheidend für die Genauigkeit und Zuverlässigkeit der Temperaturregelung ist die Genauigkeit und Zuverlässigkeit des Temperatursensors. Im Inneren eines Inkubators erfährt der Temperatursensor während seiner Lebensdauer unterschiedliche Beeinträchtigungen durch Laborproben, Reinigungsmittel, in Kombination mit unterschiedlichsten Temperaturen von bis zu 180° C, was für Sterilisationszwecke erforderlich ist. Dazu kommen Einwirkungen durch Benutzer wie Laborkräfte und Wartungspersonal, die regelmäßig das Kammerinnere manipulieren. Die Funktionsfähigkeit des Temperatursensors kann durch die genannten Einflüsse leiden. Um die Zuverlässigkeit des Temperatursensors zu gewährleisten, kann man in regelmäßigen Zeitabständen eine Kalibrierung des Temperatursensors durchführen, was aber einen gewissen Aufwand erfordert. Es besteht deshalb ein Bedarf an Maßnahmen, die den zuverlässigen und effizienten Betrieb einer Labortemperiervorrichtung begünstigen.

US 6,518,059 B1 offenbart einen Labor-Mikrotiterplatten-Inkubator mit einem Gehäuse mit einer darin eingeschlossenen Inkubationskammer und einer Temperatursteuerungsanordnung, die die Temperatur innerhalb des Inkubators gleichmäßig in einem gewünschten Bereich hält. Die Temperatursteuerungsanordnung umfasst einen Heizer, der innerhalb des Gehäuses zum Erwärmen der Kammer angeordnet ist, einen Temperatursensor und eine Steuerung.

US 6,063,619 offenbart einen Inkubator, der für eine anaerobe Umgebung mit konstanter Temperatur innerhalb eines Inkubationsgefäßes sorgt und einen Inkubatorkörper, ein im Inkubatorkörper angeordnetes oben offenes Inkubationsgefäß und ein äußeres zylindrisches Gehäuse aus Aluminium und ein inneres zylindrisches Gehäuse aus rostfreiem Stahl aufweist. Ein planarer Heizer ist in engem Kontakt mit der Außenfläche des äußeren zylindrischen Gehäuses angeordnet, und ein Temperatursensor ist in der Wand des äußeren zylindrischen Gehäuses montiert. Die Erwärmung des Inkubationsgefäßes durch den Heizer wird durch einen an der Vorderseite des Inkubatorkörpers vorgesehenen Schalter gesteuert. Aus dem Dokument GB2438683 A ist eine Temperiervorrichtung bekannt, bei der ein Temperatursensor, der im thermischen Kontakt zu einer Außenseite der Kammer angebracht ist, zur Bestimmung einer Kammerinnentemperatur genutzt wird.

Der vorliegenden Erfindung liegt deshalb die Aufgabe zugrunde, eine verbesserte Labortemperiervorrichtung bereitzustellen, die sich zuverlässig und effizient betreiben lässt und ein Verfahren anzugeben, gemäß dem sich diese Labortemperiervorrichtung zuverlässig und effizient betreiben lässt.

Die Erfindung löst diese Aufgabe durch die Labortemperiervorrichtung gemäß Anspruch 1 und die Verfahren gemäß der Ansprüche 6, 9 und 15. Bevorzugte Ausgestaltungen sind insbesondere Gegenstände der Unteransprüche.

Die erfindungsgemäße Labortemperiervorrichtung dient dem Temperieren von Laborproben bei einer Zieltemperatur $T_{ziel}$, und wird durch die Merkmale des Anspruchs 1 definiert.

Bei der erfindungsgemäßen Labortemperiervorrichtung erfolgt die Einstellung der Temperatur im Innenraum der Kammer durch Regelung der Temperiereinrichtung(en) mittels der Messung eines außerhalb der Kammer positionierten Temperatursensors. Durch diese Anordnung liegt der Temperatursensor in einem Bereich, der von der Umwelt und vom Kammerinneren abgeschirmt ist. Durch die Isolierungseinrichtung, insbesondere ein Außengehäuse, liegt der für die Einstellung der Temperatur im Innenraum relevante Temperatursensor vollkommen geschützt. Dadurch erhöht sich die zuverlässige Betriebsdauer des Temperatursensors und die Labortemperiervorrichtung lässt sich effizienter betreiben. Der Begriff "temperieren" bezeichnet allgemein das Einstellen der Temperatur, was durch Heizen und/oder Kühlen erfolgen kann und was eine Regelungseinrichtung oder eine andere Steuerung der Temperatureinstellung implizieren kann. Die Temperatur $T_{ziel}$ kann insbesondere vom Benutzer auswählbar sein.

**EP 3 539 660 B1**

Diesem erfindungsgemäßen Konzept liegen Messungen an Prototypen zugrunde, aus denen sich überraschend ergab, dass für die zuverlässige Einstellung der Temperatur im Kammerinnenraum einer mittels einer Isolierungsvorrichtung nach außen isolierten Kammer ein Temperatursensor im Kammerinnenraum nicht benötigt wird. Vielmehr ergab sich, dass es hinreichend ist, mittels eines außen an der Kammer gelegenen Temperatursensors und der äußeren Temperiereinrichtungen eine Regelung durchzuführen. Die Temperatur im Kammerinnenraum folgt bei solchen Labortemperiervorrichtungen, die üblicher Weise bei Außentemperaturen von 18 °C bis 28 °C betrieben werden, der an der Außenseite der Kammer geregelten Temperatur gemäß einer festen Gesetzmäßigkeit, die nur von der jeweiligen Labortemperiervorrichtung abhängt. Insbesondere hängt diese Gesetzmäßigkeit nur vom individuellen Aufbau der Labortemperiervorrichtung ab -dies wird als "gerätespezifisch" bezeichnet-, oder, sie hängt, bei einem stets gleichen Aufbau von Labortemperiervorrichtungen, nur vom Typ der Labortemperiervorrichtung ab - dies wird als "vorrichtungsspezifisch" bezeichnet.

Bei einer vorrichtungsspezifischen Gesetzmäßigkeit oder Eigenschaft unterscheiden sich baugleiche Labortemperiervorrichtungen hinsichtlich dieser Gesetzmäßigkeit oder Eigenschaft nicht individuell, die feste Gesetzmäßigkeit lässt sich dann auf alle baugleichen Labortemperiervorrichtungen, also einen Typ von Labortemperiervorrichtungen anwenden. Die feste Gesetzmäßigkeit liegt vorliegend in einem konstanten, gerätespezifischen oder vorrichtungsspezifischen Wert $T_{x0}$ : die Temperatur $T_K$ im Kammerinnenraum ist mit der außen gemessenen Temperatur $T_{ak}$ korreliert, die außen gemessenen Temperatur $T_{ak}$ folgt der Temperatur $T_K$ im Kammerinnenraum, und im stationären Gleichgewicht der Wärmeflüsse der Labortemperiervorrichtung ist immer derselbe Wert $T_{ak} = T_{x0}$ erreicht. Um die Temperatur $T_K$ im Kammerinnenraum zuverlässig und genau einzustellen, muss also nur der außen gelegene Temperatursensor auf $T_{ak} = T_{x0}$ geregelt werden. Der Wert $T_{x0}$ ist für jede gewünschte Zieltemperatur $T_K = T_{Ziel}$ im Kammerinnenraum unterschiedlich. Das Wertepaar ($T_{Ziel}$ ; $T_{x0}$) bzw. mehrere dieser Werte werden anhand einer Labortemperiervorrichtung vorab ermittelt, was nachfolgend noch erläutert wird, und können dann, im Falle einer gerätespezifischen Gesetzmäßigkeit, in der individuellen Labortemperiervorrichtung oder, im Falle einer vorrichtungsspezifischen Gesetzmäßigkeit, in allen baugleichen Labortemperiervorrichtungen verwendet werden.

Eine gerätespezifische Festlegung von $T_{x0}$ oder einer anderen Eigenschaft bietet den Vorteil, dass nicht alle Geräte einer Fertigungsserie baugleich ausgestattet sein müssen, was eine größere Flexibilität bei der Komponentenauswahl gewährt.

Vorzugsweise weist die Labortemperiervorrichtung keinen Temperatursensor auf, der die Temperatur im Kammerinnenraum misst. Dadurch kann ein Temperatursensor eingespart werden und dessen Wartung wird vermieden. Die Kosteneffizienz der Labortemperiervorrichtung ist dadurch verbessert.

Vorzugsweise weist die Labortemperiervorrichtung keine Regelungseinrichtung auf, mit der die Temperatur $T_K$ im Kammerinnenraum geregelt wird. Vorzugsweise weist die Labortemperiervorrichtung keine Regelungseinrichtung auf, die einen die Temperatur $T_K$ im Kammerinnenraum messenden Temperatursensor beinhaltet, der insbesondere als Messglied dieser Regeleinrichtung arbeitet. Insbesondere wird die Temperatur $T_K$ im Kammerinnenraum nur als Folge der Temperaturregelung $T_{ak} = T_{x0}$ auf den gewünschten Wert eingestellt, als zwangsläufige Folge der genannten inhärenten Eigenschaften bzw. der genannten Gesetzmäßigkeit für die Labortemperiervorrichtung. Mit anderen Worten, erfindungsgemäß wird die Temperatur $T_{ak}$ des Messsensors an der Außenseite der Kammer durch Betreiben der mindestens einen Temperiereinrichtung auf die Solltemperatur $T_{x0}$ geregelt, und die Temperatur $T_K$ des Kammerinnenraums folgt dieser Regelung über den vorbestimmten gerätespezifischen oder vorrichtungsspezifischen Zusammenhang ($T_{ziel}$; $T_{x0}$) zwischen $T_{x0}$ und der gewünschten Zieltemperatur $T_K = T_{ziel}$ des Kammerinnenraums. In dem vorgenannten Sinne kann ma diese Art der Temperatureinstellung im Kammerinnenraum auch als "indirekte Regelung" der Temperatur $T_K$ im Kammerinnenraum bezeichnen, auch wenn es sich nicht um eine Temperaturregelung von $T_K$ im herkömmlichen Sinn handelt.

Vorzugsweise weist die elektrische Steuereinrichtung eine Datenspeichereinrichtung auf. In dieser ist vorzugsweise der mindestens eine vorbestimmte Wert $T_{x0}$ gespeichert, der zur Temperaturregelung $T_{ak} = T_{x0}$ aus der Datenspeichereinrichtung entnommen wird. Der Wert $T_{x0}$ kann aber nicht nur digital, sondern alternativ auch analogelektronisch bereitgestellt werden.

Die elektronische Steuereinrichtung ist dazu eingerichtet, dass die Temperiereinrichtung während der Temperaturregelung $T_{ak} = T_{x0}$ in Abhängigkeit von der Zeit t mit der elektrischen Leistung $P_{temp}(t)$ betrieben wird. Insbesondere kann die Labortemperiervorrichtung dazu eingerichtet sein, dass die Temperiereinrichtung mittels einer Pulsweitenmodulation (PWM) des Stroms betrieben wird. Die Leistung wird dann insbesondere durch den Tastgrad der PWM bestimmt, da die Amplitude des Stroms vorzugsweise konstant ist.

Die elektronische Steuereinrichtung ist dazu eingerichtet, dass die elektrische Steuereinrichtung diese Leistung $P_{temp}(t)$ und den zeitabhängigen Messwert $T_{ak}(t)$ erfasst. Die elektronische Steuereinrichtung weist eine Datenverarbeitungseinrichtung auf, insbesondere einen Computerprozessor. Die Datenverarbeitungseinrichtung ist dazu eingerichtet, eine Größe $T_{Kb}$ als Temperatur zu ermitteln bzw. zu berechnen, die die Temperatur $T_K$ des Kammerinnenraums beschreibt bzw. abschätzt. Dies erfolgt insbesondere, indem die Datenverarbeitungseinrichtung die Größe $T_{Kb}$ als von mindestens einem anderen Parameter abhängige Größe bestimmt, insbesondere mittels einer Ermittlungsvorschrift

3

bzw. mindestens einer Gleichung. Die Datenverarbeitungseinrichtung ist dazu eingerichtet, die Temperatur $T_{Kb}$ in Abhängigkeit von der Leistung $P_{temp}(t)$ und der gemessenen Temperatur $T_{ak}(t)$ zu berechnen. Zum Zwecke der Ermittlung von $T_{Kb}$ ist die Datenverarbeitungseinrichtung vorzugsweise entsprechend programmiert. Insbesondere weist die elektronische Steuereinrichtung bzw. die Datenverarbeitungseinrichtung ein Computerprogramm auf, das $T_{Kb}$ ermittelt, wenn es von der Steuereinrichtung bzw. der Datenverarbeitungseinrichtung ausgeführt wird. Das Computerprogramm kann in einer Datenspeichereinrichtung der Labortemperiervorrichtung gespeichert sein. Die Steuereinrichtung bzw. die Datenverarbeitungseinrichtung ist insbesondere dazu eingerichtet, eines der erfindungsgemäßen Verfahren auszuführen, und/oder mindestens einen, mehrere oder alle Verfahrensschritte eines der erfindungsgemäßen Verfahren auszuführen.

Vorzugsweise weist die Labortemperiervorrichtung einen Kammertürsensor auf, der das Öffnen und Schließen der Kammertüre erfasst. Im Fall einer Labortemperiervorrichtung, die ein äußeres Gehäuse und eine Gehäusetüre aufweist, wie das meist bei Inkubatoren der Fall ist, ist vorzugsweise ein Gehäusetürsensor vorgesehen, der das Öffnen und Schließen der Gehäusetüre erfasst. Vorzugsweise weist die Labortemperiervorrichtung ein Außengehäuse mit einer Gehäusetüre auf, die im geöffneten Zustand dem Benutzer durch die geöffnete Kammertüre den Zugang zum Kammerinnenraum ermöglicht, und insbesondere einen Gehäusetürsensor, der das Öffnen und Schließen der Gehäusetüre erfasst. Die elektrische Steuereinrichtung ist vorzugsweise dazu eingerichtet ist, das Öffnen und Schließen der Kammertüre oder der Gehäusetüre zeitabhängig zu erfassen und die Temperatur $T_{Kb}$ als Temperatur des Kammerinnenraums auch in Abhängigkeit von den Zeitpunkten des Öffnens und Schließens der Kammertüre oder der Gehäusetüre zu berechnen. Dadurch wird das Problem gelöst, dass die Temperatur des Kammerinnenraums bekannt sein soll, obwohl diese bei der Erfindung nicht zwangsläufig gemessen wird.

Vorzugsweise ist die elektrische Steuereinrichtung dazu eingerichtet, nach dem Öffnen und Schließen der Kammertüre oder der Gehäusetüre der Labortemperiervorrichtung eine Temperatur $T_{Kb}(t)$ in Abhängigkeit von der Zeit t als Temperatur des Kammerinnenraums gemäß der bevorzugt verwendeten Gleichung

$$T_{Kb}(t) = T_{ak}(t) + T_{offset} - (P_{temp}(t) - P_{Basis}(t)) * F \quad \text{(Gleichung 1)}$$

zu berechnen, wobei $T_{offset}$ definiert ist durch $T_{offset} = T_{x0} - T_{ziel}$, $P_{Basis}(t)$ ist die vor dem Öffnen der Kammertüre oder Gehäusetüre im stationären Zustand bei gegebener Umgebungstemperatur von der Temperiereinrichtung, oder mehreren Temperiereinrichtungen, aufgebrachte Leistung, und F ist ein Normierungsfaktor. Dessen Ermittlung wird nachfolgend noch beschrieben. Der Wert F ist vorzugsweise vorrichtungsspezifisch und wird vorzugsweise digital in einer Datenspeichereinrichtung der elektrischen Steuereinrichtung gespeichert oder analogelektronisch bereitgestellt. Der Wert F kann aber auch gerätespezifisch sein.

$P_{Basis}(t)$ ist insbesondere ein gleitender Mittelwert aus zeitlich möglicherweise variierenden Werten $P_{Basis}(t)$. Durch eine solche Mittelwertbildung wird eine größere Präzision erreicht. Die elektrische Steuereinrichtung ist vorzugsweise dazu eingerichtet, eine Vielzahl von Werten $P_{Basis}(t)$ zeitabhängig zu speichern. Insbesondere ist die elektrische Steuereinrichtung vorzugsweise dazu eingerichtet, das Öffnen und Schließen der Kammertüre oder Gehäusetüre, abgekürzt als ZT(t) ("Zustand Türe"), zeitabhängig zu erfassen und zu speichern. Die elektrische Steuereinrichtung ist vorzugsweise dazu eingerichtet, durch die Kombination der Informationen $P_{Basis}(t)$ und ZT(t) den mittleren Wert der Leistung vor dem Öffnen der Türe im stationären Gleichgewicht zu ermitteln, insbesondere als gleitenden Mittelwert von Daten $P_{Basis}(t)$, insbesondere als gleitenden Mittelwert von Tastgrad-Werten, die abgekürzt bezeichnet werden als PWM, zu ermitteln. Im stationären Gleichgewicht der Wärmeflüsse der Labortemperiervorrichtung hängt $P_{Basis}(t)$ von der Außentemperatur ab, die üblicherweise zwischen 18 °C und 28 °C beträgt.

Für einen bestimmten Typ einer Labortemperiervorrichtung wird $T_{x0}$ vorzugsweise vom Hersteller bestimmt. In einem im thermischen Gleichgewicht der Kammer durchgeführten Kalibrierverfahren wird vorzugsweise $T_{x0}$ mittels des äußeren Temperatursensors $T_{ak}$ bei der vorbestimmten Kammerinnenraumtemperatur $T_K = T_{ziel}$ an der Labortemperiervorrichtung oder einer baugleichen Labortemperiervorrichtung gemessen. $T_K = T_{ziel}$ wird bei dem Kalibrierverfahren vorzugsweise verifiziert eingestellt, also mit kalibriertem Sensor eingestellt, insbesondere geregelt, indem ein zu Kalibrierzwecken in den Kammerinnenraum eingesetzter mobiler -kalibrierter- Temperatursensor mit der elektrischen Steuereinrichtung der Labortemperiervorrichtung verbunden wird, so dass diese einen Regelkreis ausführt, bei dem der mobile Temperatursensor $T_{innen}$ im Kammerinnenraum als Messglied des Regelkreises arbeitet und die Temperiereinrichtung(en) als Stellglied des Regelkreises.

Für einen bestimmten Typ einer Labortemperiervorrichtung wird der Normierungsfaktor F vorzugsweise vom Hersteller bestimmt. Dazu wird die Labortemperiervorrichtung in einer Umgebung mit einer bekannten Außentemperatur $T_{umgebung}$ platziert, wobei beispielsweise $T_{umgebung} = 18$ °C. Die Kammerinnenraumtemperatur $T_K$ wird auf einen Wert $T_K = T_{ziel\_0}$ eingestellt, z.B. $T_{ziel\_0} = 50$ °C. Insbesondere ist $T_{umgebung}$ nicht gleich $T_{ziel\_0}$ ($T_{umgebung} <> T_{ziel\_0}$). Dazu wird ein in den Kammerinnenraum eingesetzter mobiler Temperatursensor $T_{innen}$ mit der elektrischen Steuereinrichtung der Labortemperiervorrichtung verbunden, so dass diese einen Regelkreis ausführt, bei dem der mobile Temperatursensor $T_{innen}$ im Kammerinnenraum als Messglied des Regelkreises arbeitet und die Temperiereinrichtung(en) als Stellglied des Regelkreises. Die Regelung erfolgt bei geschlossener Kammertüre und/oder Gehäusetüre (falls Inkubator, dann Gehäusetüresensor). Zu einem bestimmten Zeitpunkt t0 wird die Kammertüre geöffnet, und zu einem späteren Zeitpunkt t1, der z.B. zwischen 1 und 10 Minuten nach t0 liegen kann, wieder geschlossen. Vor und während dem Öffnen und Schließen und danach wird die Kammerinnenraumtemperatur $T_{innen}(t)$ gemessen und zeitabhängig gespeichert. Danach wird vorzugsweise die oben genannte "Gleichung 1" oder eine andere Gleichung als Fitkurve verwendet, um mittels Ausgleichsrechnung zwischen der Fitkurve und den Messwerten $T_{innen}(t)$ den Faktor F zu ermitteln, wobei die Ausgleichsrechnung insbesondere, vorzugsweise ausschließlich, den Zeitbereich zwischen dem Öffnen und Schließen der Kammertüre oder Gehäusetüre berücksichtigt. Der so ermittelte Faktor F wird vorzugsweise in allen weiteren Labortemperiervorrichtungen desselben Typs gespeichert und zur Schätzung bzw. Berechnung der Kammerinnenraumtemperatur verwendet. Die Speicherung erfolgt jeweils vorzugsweise in einer Datenspeichereinrichtung der elektrischen Steuereinrichtung der Labortemperiervorrichtung.

Vorzugsweise weist die Labortemperiervorrichtung eine Benutzerschnittstelleneinrichtung mit einer Anzeigeneinrichtung auf, die dazu eingerichtet ist, auf der Anzeigeneinrichtung eine visuelle Anzeige auszugeben. Eine Benutzerschnittstelleneinrichtung kann Bestandteil einer Labortemperiervorrichtung, insbesondere eines Inkubators sein, oder kann ein Modul sein, das mit mehreren Labortemperiervorrichtungen desselben Typs verwendbar ist. Die elektrische Steuereinrichtung der Labortemperiervorrichtung oder eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeigeneinrichtung bzw. ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung der Labortemperiervorrichtung bzw. des Inkubators auszutauschen. Die Labortemperiervorrichtung und/oder deren Steuereinrichtung und die Benutzerschnittstelleneinrichtung können insbesondere dazu eingerichtet sein, dass der Benutzer die gewünschte Zieltemperatur $T_{ziel}$ über die Benutzerschnittstelleneinrichtung auswählen oder eingeben kann, und dass insbesondere diese so eingegebene Zieltemperatur $T_{ziel}$ in einer Datenspeichereinrichtung der Labortemperiervorrichtung gespeichert wird.

Vorzugsweise ist die elektrische Steuereinrichtung dazu eingerichtet, auf der Anzeigeneinrichtung den berechneten Wert $T_{Kb}(t)$ auszugeben und diesen in vorgegebenen Zeitabständen zu aktualisieren, insbesondere wenn die fortlaufende Neuberechnung eine Änderung des Wertes $T_{Kb}(t)$ ergeben hat.

Die Erfindung betrifft auch ein Verfahren zum Einstellen einer Zieltemperatur $T_{ziel}$ in der Kammer einer erfindungsgemäßen Labortemperiervorrichtung, aufweisend die Schritte:

- Bestimmen des vorrichtungsspezifischen Wertes $T_{x0}$, insbesondere gemäß dem oben genannten Verfahren, wobei $T_{x0}$ so vorbestimmt wird, dass sich aus der Temperaturregelung $T_{ak} = T_{x0}$ die Einstellung der Temperatur $T_K$ im Kammerinnenraum auf $T_K = T_{ziel}$ ergibt.
- Regelung der Außentemperatur $T_{ak}$ auf $T_{ak} = T_{x0}$ , wodurch sich die Einstellung der Temperatur $T_K$ im Kammerinnenraum auf $T_K = T_{ziel}$ ergibt.

Die erfindungsgemäße Labortemperiervorrichtung, insbesondere deren Steuereinrichtung, ist vorzugsweise dazu eingerichtet, das zuvor genannte Verfahren zum Einstellen einer Zieltemperatur $T_{ziel}$ in der Kammer der Labortemperiervorrichtung auszuführen, insbesondere mittels einer zu diesem Zweck programmierten Datenverarbeitungseinrichtung der Steuereinrichtung, die insbesondere zur Ausführung eines Computerprogramms eingerichtet ist, welches das genannte Verfahren umsetzt.

Die Erfindung betrifft auch ein Verfahren zur Schätzung der Temperatur $T_K$ im Innenraum der Kammer einer erfindungsgemäßen Labortemperiervorrichtung, aufweisend die Schritte:

- Bestimmen des vorrichtungsspezifischen Wertes $T_{x0}$, insbesondere gemäß dem oben genannten Verfahren, wobei $T_{x0}$ so vorbestimmt wird, dass sich aus der Temperaturregelung $T_{ak} = T_{x0}$ die Einstellung der Temperatur $T_K$ im Kammerinnenraum auf $T_K = T_{ziel}$ ergibt;
- Bestimmen des vorrichtungsspezifischen Wertes F, insbesondere gemäß dem oben genannten Verfahren;
- insbesondere: Einstellung der Temperatur $T_K$ im Kammerinnenraum auf $T_K = T_{ziel}$ , durch die Temperaturregelung $T_{ak} = T_{x0}$ ;

- insbesondere: Öffnen und Schließen der Kammertüre und/oder Gehäusetüre der Labortemperiervorrichtung;
- Schätzung des Wertes $T_K$ im Innenraum der Kammer durch Berechnung, so dass

$$T_K = T_{Kb} = T_{Kb}(t) = T_{ak}(t) + T_{offset} - (P_{temp}(t) - P_{Basis}(t)) * F$$

wobei $T_{offset}$ definiert ist durch $T_{offset} = T_{x0} - T_{ziel}$, $P_{Basis}(t)$ ist die vor dem Öffnen der Kammertüre oder Gehäusetüre im stationären Zustand bei gegebener Umgebungstemperatur von der Temperiereinrichtung aufgebrachte Leistung, $P_{temp}(t)$ ist die fortlaufend von der Temperiereinrichtung abgerufene elektrische Leistung, die für die Temperaturregelung mit dem Temperatursensor $T_{ak}$ benötigt wird. Der Faktor F hat die Einheit "Kelvin", wenn die Leistungsgrößen $P_{temp}(t)$, $P_{Basis}(t)$ als Tastgrad einer PWM bei bekannter Amplitude angegeben werden.

Die erfindungsgemäße Labortemperiervorrichtung, insbesondere deren Steuereinrichtung, ist vorzugsweise dazu eingerichtet, das zuvor genannte Verfahren zur Schätzung der Temperatur $T_K$ im Innenraum der Kammer der Labortemperiervorrichtung auszuführen, insbesondere mittels einer zu diesem Zweck programmierten Datenverarbeitungseinrichtung der Steuereinrichtung, die insbesondere zur Ausführung eines Computerprogramms eingerichtet ist, welches das genannte Verfahren umsetzt.

Die Labortemperiervorrichtung zum Temperieren von Laborproben ist insbesondere ein Temperierschrank zum Lagern von Laborproben. Solche Geräte werden elektrisch betrieben und weisen einen Spannungsanschluss auf. Die erfindungsgemäße Labortemperiervorrichtung kann aber auch dazu eingerichtet sein, Laborproben nicht nur zu temperieren sondern auch eine andere Behandlung an mindestens einer Laborprobe durchzuführen. Die Labortemperiervorrichtung kann zur Behandlung mindestens einer Laborprobe eingerichtet sein. Die Labortemperiervorrichtung kann insbesondere ein Laborautomat sein, der zur automatisierten Handhabung mindestens einer flüssigen Laborprobe eingerichtet ist. Solche Laborautomaten sind auch als "liquid handling"-Automaten bekannt. Die Kammer eines Laborautomaten kann der Arbeitsraum sein, der von der Umgebung getrennt ist und in dem die mindestens eine Laborprobe durch eine automatisierte Behandlungseinrichtung, insbesondere einen automatisch steuerbaren Pipettierroboter, transportiert und/oder dosiert werden kann.

Der Temperierschrank temperiert die Laborproben, das heißt, er hält das Gehäuseinnere und damit die dort lagernden Laborproben im Rahmen von Toleranzen durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einer als Klimaschrank ausgebildeten Laborschrankvorrichtung wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine CO2, O2 und/oder N2-Konzentration. Ein solcher Klimaschrank ist beispielsweise ein Inkubator für aus lebenden Zellkulturen bestehende Laborproben.

Die Labortemperiervorrichtung weist vorzugsweise ein Gehäuse auf. Das Gehäuse ist vorzugsweise eine äußeres Gehäuse, dessen Gehäusewände mit der Umgebung in Kontakt stehen. Die Gehäusetüre kann entsprechend eine äußere Gehäusetüre sein, die in der Verschlussposition an die Umgebung grenzt.

Die Gehäusetüre weist insbesondere eine Scharniereinrichtung auf, welche die Gehäusetüre schwenkbar mit dem Gehäuse verbindet. Eine solche Schwenktüre wird durch eine Rotation zwischen einer geöffneten Position und der Verschlussposition bewegt. Die Scharniereinrichtung kann insbesondere an der - im bestimmungsgemäßen Gebrauch der Laborschrankvorrichtung - vertikal orientierten Außenkante eines quaderförmigen Gehäuses liegen, welche an die Gehäuseöffnung angrenzt. Die Bodenplatte eines quaderförmigen Gehäuses ist im bestimmungsgemäßen Gebrauch der Laborschrankvorrichtung horizontal angeordnet, die Seitenwände des Gehäuses sind insbesondere vertikal angeordnet, und die Deckplatte des Gehäuses ist insbesondere der Bodenplatte gegenüberliegend horizontal angeordnet.

Die Kammertüre oder Gehäusetüre kann aber auch eine Schiebetüre sein, die durch eine translatorische Bewegung zwischen einer geöffneten Position und der Verschlussposition bewegt wird. Auch eine gemischt schwenkende/translatorische Bewegung der Kammertüre oder Gehäusetüre ist möglich.

Die Datenverarbeitungseinrichtung ist vorzugsweise Bestandteil der elektrischen Steuereinrichtung, die Funktionen der Labortemperiervorrichtung steuert. Die Funktionen der Steuereinrichtung sind insbesondere durch elektronische Schaltkreise implementiert. Die Steuereinrichtung kann eine Recheneinheit (CPU) zum Verarbeiten von Daten und/oder einen Mikroprozessor aufweisen, der die Datenverarbeitungseinrichtung beinhalten kann. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Die Funktionen des Inkubators und/oder der Steuereinrichtung können in Verfahrensschritten beschrieben werden. Sie können als Bestandteile des Steuerungsprogramms realisiert sein, insbesondere als Unterprogramme des Steuerungsprogramms.

Vorzugsweise ist die Labortemperiervorrichtung ein Inkubator. Der Inkubator ist ein Labor-Inkubator und damit ein Gerät, mit dem kontrollierte Klimabedingungen für verschiedene biologische Entwicklungs- und Wachstumsprozesse geschaffen und erhalten werden können. Er dient insbesondere der Schaffung und Erhaltung eines Mikroklimas mit geregelten Gas-, und/oder Luftfeuchtigkeits- und/oder TemperaturBedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine als Heiz- und/oder Kühleinrichtung ausgeführte Temperiereinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO2 und/oder des O2 und/oder des N2-Gehalts Gehalts des Gases und/oder eine Einstelleinrichtung zur Einstellung der Luftfeuchtigkeit in der Inkubatorkammer des Inkubators.

Der Inkubator weist insbesondere die Inkubatorkammer (= Kammer) auf, ferner vorzugsweise eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. CO2-Inkubatoren dienen insbesondere der Kultivierung tierischer bzw. humaner Zellen. Inkubatoren können Wendevorrichtungen zum Wenden des mindestens einen Zellkulturbehälter und/oder eine Schütteleinrichtung zum Schütteln bzw. Bewegen der des mindestens einen Zellkulturbehälter aufweisen.

Die Steuerungseinrichtung kann dazu eingerichtet sein, dass ein Programmparameter oder ein Steuerungsparameter des Inkubators automatisch in Abhängigkeit von anderen Daten gewählt wird, insbesondere ZT(t). Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, die relative Gaskonzentration von O2- und/oder CO2 und/oder N2 im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Es sind ein oder mehrere Temperatursensoren vorgesehen, die insbesondere im thermischen Kontakt mit der Außenseite, insbesondere Kammerwand, insbesondere oberer Kammerwand angeordnet sind. Zur Ausbildung des thermischen Kontakts kann der Temperatursensoren die Außenseite kontaktieren, insbesondere direkt kontaktieren oder über eine Wärmeleitungsmedium, z.B. eine Wärmeleitpaste, eine Wärmeleitfolie oder ein wärmeleitendes Bauteil. Der Temperatursensor kann gegen die Außenseite gepresst montiert sein. Auch eine Vielzahl >=10 von Temperatursensoren können vorgesehen sein. Ein Temperatursensor kann beispielsweise ein Pt 100- oder Pt 1000-Temperaturfühler sein.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen. Die Steuereinrichtung kann einen oder mehrere Regelkreise aufweisen, wobei jeder Regelkreis mindestens eine Temperiereinrichtung und insbesondere mindestens einen Temperatursensor aufweist, der insbesondere im thermischen Kontakt zu einer Außenseite der Kammer zur Erfassung einer Temperatur Taußen $T_{ak}$ angebracht ist. Im Falle mehrerer Regelkreise sind die als Stellglieder dienenden Temperiereinrichtungen und die als Messglieder dienenden Temperatursensoren insbesondere an unterschiedlichen Positionen der Kammeraußenseite angeordnet, insbesondere an verschiedenen Außenseiten der Kammer, z.B. der Deckenwand, Bodenwand, Seitenwand, Frontwand und/oder Rückwand. Im Falle mehrerer Regelkreise sind vorzugsweise dieselbe Anzahl gerätespezifischer Werte $T_{x0}$ in einer Datenspeichereinrichtung der Labortemperiervorrichtung gespeichert, wobei jedem Regelkreis ein eigener Wert $T_{x0}$ zugeordnet ist, auf den die Temperatur des jeweiligen Temperatursensors geregelt wird, um im Kammerinnenraum die Zieltemperatur $T_{ziel}$ einzustellen. Vorzugsweise ist eine Anzahl N von Regelkreisen vorgesehen, deren Temperiereinrichtungen und Temperatursensoren insbesondere an unterschiedlichen Positionen der Außenwand der Kammer angeordnet sind, wobei vorzugsweise 1 < N < 10, und vorzugsweise N insbesondere ausgewählt ist aus {1, 2, 3, 4, 5, 6}. Besonders bevorzugt ist N=4.

Falls die Labortemperiervorrichtung mehrere Kammern aufweist, die voneinander mittels der Isolierungseinrichtung oder einer anderen Isoliereinrichtung thermisch getrennt sind, kann für jede Kammer auch eine eigene Zieltemperatur $T_{ziel}$ vorgesehen sein, die mittels entsprechender Regelung $T_{ak} = T_{x0}$ eingestellt wird.

Die Labortemperiervorrichtung bzw. der Inkubator kann genau eine Kammer aufweisen, kann aber auch mehrere Kammern aufweisen, deren Atmosphäre (Temperatur, relative

Gaskonzentration, Luftfeuchte) insbesondere individuell oder gesammelt einstellbar sein kann. Eine typische Größe des Inneren einer Kammer liegt zwischen 50 und 400 Litern.

Bevorzugte Ausgestaltungen der erfindungsgemäßen Labortemperiervorrichtung können insbesondere der Beschreibung eines der erfindungsgemäßen Verfahren entnommen werden. Bevorzugte Ausgestaltungen der erfindungsgemäßen Verfahren können insbesondere der Beschreibung der erfindungsgemäßen Labortemperiervorrichtungen entnommen werden.

Weitere bevorzugte Ausgestaltungen der erfindungsgemäßen Laborschrankvorrichtung lassen sich der Beschreibung der Ausführungsbeispiele gemäß den Figuren entnehmen.

Es zeigen:

Fig. 1a zeigt eine perspektivische Frontansicht eines erfindungsgemäßen Inkubators gemäß Ausführungsbeispiel.

Fig. 1b zeigt eine perspektivische Rückansicht des Inkubators der Fig. 1a.

Fig. 2 zeigt eine schematische seitliche Querschnittsansicht des Inkubators aus Fig. 1a, 1b.

Fig. 3 zeigt ein Diagramm mit der Temperatur Top_Heizkreis=$T_{ak}$ + $T_{offset}$, im Vergleich zu einer wahren Kammertemperatur, die mit einem mobilen Temperatursensor gemessen wurde.

Fig. 4 zeigt ein Diagramm, bei dem verschiedene temperaturbezogene Parameter des Inkubators gegen die Zeit aufgetragen sind, wenn die Kammer- und Gehäusetür zunächst geöffnet und dann wieder geschlossen wird.

Fig. 5 zeigt ein Diagramm, bei dem, gegen die Zeit aufgetragen, die Kurve der wahren Kammertemperatur $T_K$ gezeigt ist, die mit einem mobilen Temperatursensor gemessen wurde, und eine Ausgleichskurve, die gemäß einer Ausgleichsrechnung berechnet wurde.

Fig. 6, 7, 8 und 9 zeigen Ausführungsbeispiele erfindungsgemäßer Verfahren.

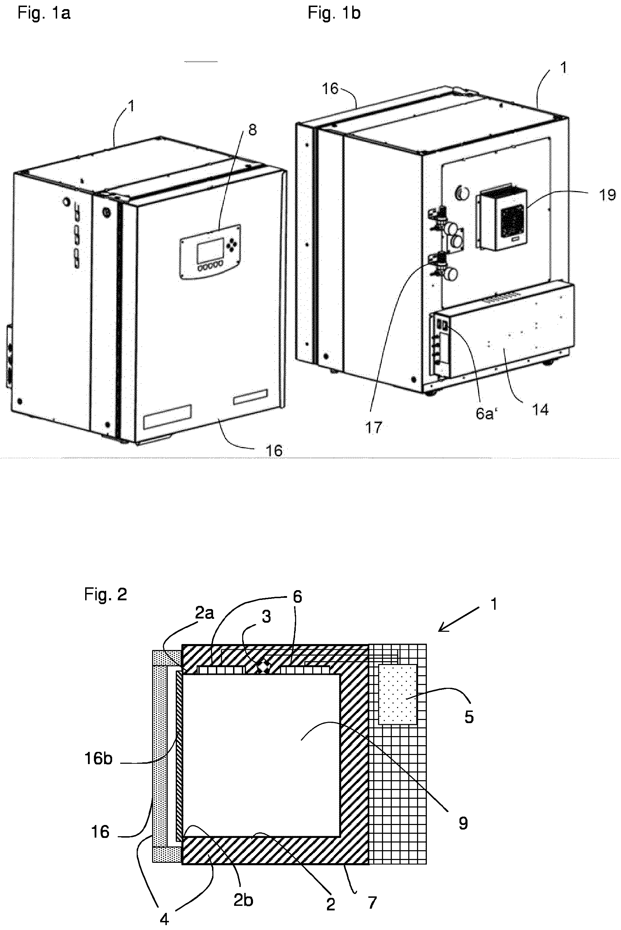

Fig. 1a zeigt die als Inkubator 1 ausgebildete Labortempereinrichtung für das Wachstum von Zellkulturen, hier ein CO2-Inkubator für das Wachstum von eukaryotischen Zellen. Der Inkubator besitzt: eine Inkubatorkammer (siehe Figur 2) zur Aufnahme mindestens eines Zellkulturbehälters, der eine darin wachsende Zellkultur enthält, einen Temperatursensor 3, der an der Außenseite der oberen Kammerwand 2a im thermischen Kontakt mit dieser befestigt ist. Der Inkubator weist auf:

* eine Kammer 2 zur Aufnahme von Laborproben im Kammerinnenraum 9 auf, deren Außenseiten durch mindestens eine Kammerwand 2a sowie eine Kammertüre 16b gebildet werden, welche eine Kammeröffnung 2b verschließt, durch die der Kammerinnenraum für den Benutzer zugänglich ist,
* eine Temperiereinrichtung 6 zum Temperieren der Kammer 2, die im thermischen Kontakt zu einer durch die obere Kammerwand 2a gebildeten Außenseite 2a der Kammer angebracht ist,
* einen Temperatursensor 3, der im thermischen Kontakt zu derselben Außenseite 2a der Kammer 2 zur Erfassung einer Temperatur $T_{ak}$ angebracht ist,
* eine Isolierungseinrichtung (4, 16), beinhaltend eine Isolationsschicht 4 und die Gehäusetüre 16, welche die Temperiereinrichtung, den Temperatursensor und die Kammer gegenüber der Umgebung thermisch isolieren, und
* eine elektrische Steuereinrichtung 5, die dazu eingerichtet ist, die Temperatur $T_{ak}$ mittels des Temperatursensors und der Temperiereinrichtung auf eine Solltemperatur mit dem Wert $T_{x0}$ zu regeln, der so vorbestimmt ist, dass sich aus der Temperaturregelung $T_{ak}$ = $T_{x0}$ die Einstellung der Temperatur $T_K$ im Kammerinnenraum auf $T_K$ = $T_{ziel}$ ergibt.

Der Inkubator 1 weist ein Display der Benutzerschnittstelleneinrichtung 8 auf, über welche die elektrische Steuereinrichtung 5 Informationen an den Benutzer ausgibt und über das der Benutzer Eingaben vornehmen kann. Zu den hier angezeigten Informationen gehört insbesondere die geschätzte Temperatur $T_{Kb}$ des Kammerinnenraums 9. Der Inkubator weist keinen Sensor auf, mit dem die Kammerinnenraumtemperatur $T_K$ mit Bordmitteln gemessen werden könnte. Die elektrische Steuereinrichtung 5 ist in einem Elektronik-Kompartiment 14 des Gehäuses 7 angeordnet, wobei das Kompartiment 14 außerhalb der zwischen Kammer 2 und Gehäuse 7 angeordneten Isolierschicht 4 auf der Rückseite des Inkubators angeordnet ist, die angeordnet ist. Am Kompartiment 14 findet sich auch die Spannungsversorgung 6a des Inkubators. Zudem sind Gasanschlüsse 17 für N2 und CO2 an der Rückseite des Inkubators vorgesehen.

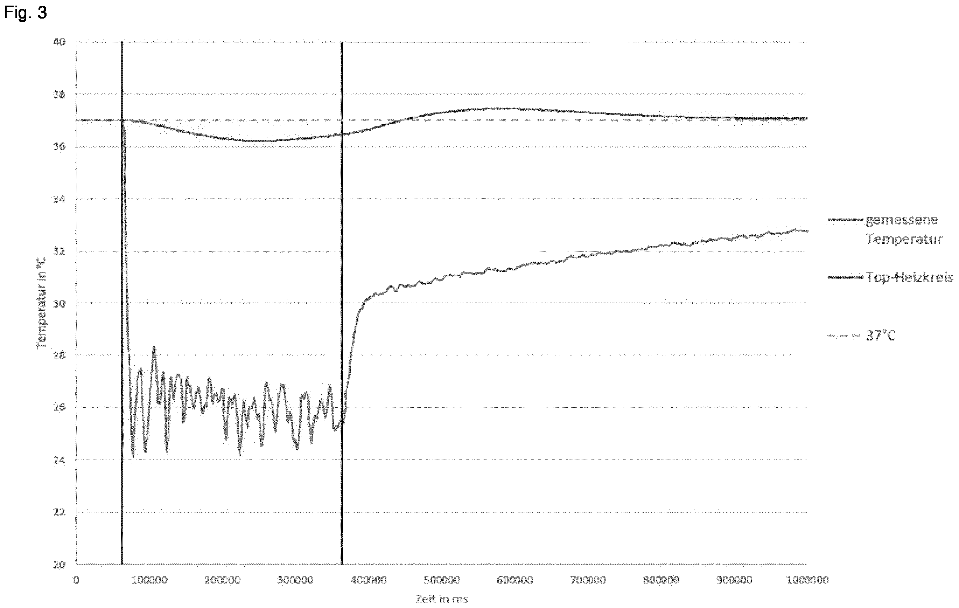

In Fig. 3 ist gezeigt, dass die im Innenraum 9 der Kammer 2 mittels verifiziertem mobilen Temperatursensor messbare Kammertemperatur $T_K$ ("gemessene Temperatur") nach dem Öffnen der Gehäusetüre 16 und Kammertüre 16b von 37 °C auf ca. 26 °C absackt, und dass sich die Kammertemperatur nach dem Schließen der Türen langsam wieder zum Ausgangswert 37 °C verändert. Dies erfolgt, indem die Temperatur $T_{ak}$ ("Top-Heizkreis") des an der Außenseite angeordneten Temperatursensors 3 auf den vorbestimmten, hier gerätespezifischen Wert $T_{x0}$ geregelt wird. Dieser Wert $T_{x0}$ kann von der Zieltemperatur abweichen und wird diese in der Regel tun, muss aber nicht. Im konkreten Fall des beispielhaften Inkubators lag dieser Wert unterhalb von 37 °C, was daran liegt, dass der verwendete Temperatursensor, der $T_{ak}$ misst, nicht kalibriert ist - eine solche Sensorkalibrierung ist nicht erforderlich, da die Kalibrierung der Temperaturgenauigkeit der Labortempiervorrichtung über die Vorbestimmung des Werts $T_{x0}$ erfolgt. Man sieht, dass der Wert Top_Heizkreis=$T_{ak}$ + $T_{offset}$ nur wenig schwankt. Dennoch ist dieser Wert, gemäß der erfindungsgemäßen Lehre, fest mit der Kammertemperatur $T_K$ verknüpft.

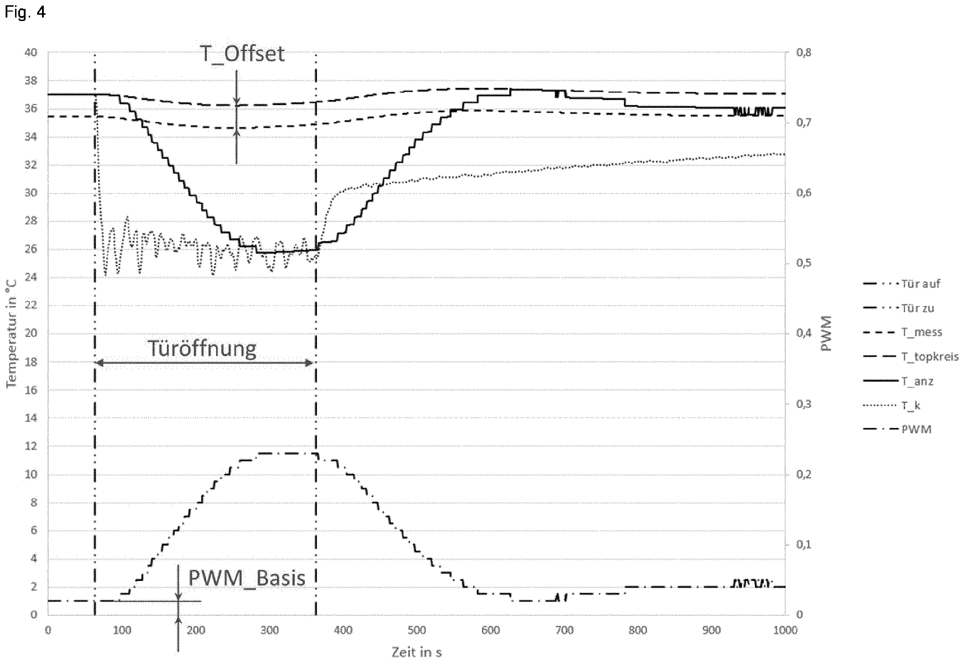

Fig. 4 zeigt ein Diagramm, bei dem verschiedene temperaturbezogene Parameter des Inkubators gegen die Zeit aufgetragen sind, wenn die Kammer- und Gehäusetür zunächst geöffnet (erste vertikale Linie bei $t_0$=70s) und dann wieder geschlossen (zweite vertikale Linie bei $t_1$=370s) wird. Zum Zeitpunkt $t_0$=70s wird ein die Leistungsaufnahme der Temperiereinrichtungen 6 (Heizkreis) charakterisierender Leistungsbasiswert $P_{Basis}(t)$ ("PWM_Basis") erfasst, der als gleitender Mittelwert aus hier 30 -zu diesem Zeitpunkt bereits vergangenen, aber in einem Datenspeicher der elektrischen Steuereinrichtung gespeicherten- Werten für $P_{Basis}(t)$ ermittelt wird. Die Kurve $P_{Basis}(t)$ der Leistungsaufnahme der Temperaturregelung wird als "PWM" bezeichnet, die Skala dafür befindet sich auf der rechten Ordinatenachse. Es handelt sich um Werte um die 0,01 bis 0,3, die den zeitabhängigen Tastgrad der mittels Pulsweitenmodulation gesteuerten Stromversorgung der Temperiereinrichtung angeben. Die wahre Kammertemperatur $T_K$ wird zur Erläuterung und zum Verständnis auch dargestellt, gemessen mittels verifiziertem mobilen Temperatursensor, platziert zu Prüfzwecken im Innenraum 9 des Inkubators. Die Temperatur des Temperatursensors 3 an der Außenseite der Kammerwand 2a ist als Kurve "T_mess" gezeigt. Die Kurve "T_topkreis" ergibt sich aus einem Wert $T_{offset}$ ("T _offset") als T_topkreis = $T_{ak}(t)$ + $T_{offset}$. Die Kurve $T_{Kb}(t)$ ist ebenfalls eingetragen, als "T_anz". Die gemäß dieser Kurve variierenden Temperaturwerte $T_{Kb}$ werden als berechnete Kammertemperatur im Display 8 angezeigt. Es erfolgt hier die Schätzung des Wertes $T_K$ im Innenraum 9 der Kammer durch Berechnung, gemäß der in diesem Beispiel verwendeten Gleichung 1:

$$T_K = T_{Kb} = T_{Kb}(t) = T_{ak}(t) + T_{offset} - (P_{temp}(t) - P_{Basis}(t)) * F.$$

Hierbei ist der vorrichtungsspezifische Wert $T_{x0} = T_{ziel} + T_{offset}$. Hier ist $T_{ziel}$ = 37 °C.

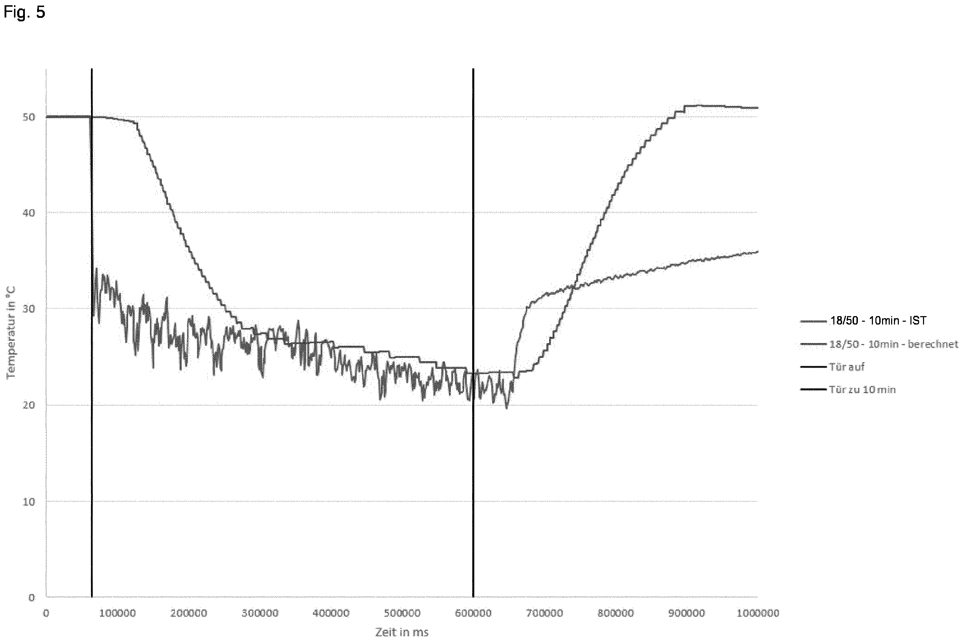

Fig. 5 zeigt ein Diagramm (Temperatur gegen die Zeit), um die Ermittlung des vorrichtungsspezifischen Wertes F zu erläutern. Aufgetragen ist wieder die wahre Kammertemperatur $T_K$ ("18/50-10min-IST"), gemessen mittels verifiziertem mobilen Temperatursensor, platziert zu Prüfzwecken im Innenraum 9 des Inkubators. Die Umgebungstemperatur lag bei 18 °C, die Kammerinnenraumtemperatur war auf 50 °C eingestellt. Das Öffnen der Tür hat ein Absacken der Kammertemperatur $T_K$ zur Folge. An die Kurve $T_K$ (t) ("18/50-10min-berechnet") wird im Zeitbereich zwischen dem Öffnen und Schließen der Türe(n) die Ausgleichskurve ("Fitkurve") durch Parametervariation optimal angelegt (Ausgleichsrechnung). Die Fitkurve folgt in diesem Beispiel der Gleichung 1; es lässt sich daraus der Faktor F ableiten, der für den Inkubator festgelegt ist. Beide vorrichtungsspezifischen Werte $T_{x0}$ und F werden in einer Datenspeichereinrichtung abgelegt. Mittels $T_{x0}$ und der Temperaturregelung des Temperatursensors $T_{ak}$ kann die Temperatur im Kammerinnenraum eingestellt werden. Mittels des Normierungsfaktors F kann der nach einer Türöffnung erfolgende Temperaturverlauf im Kammerinnenraum abgeschätzt werden.

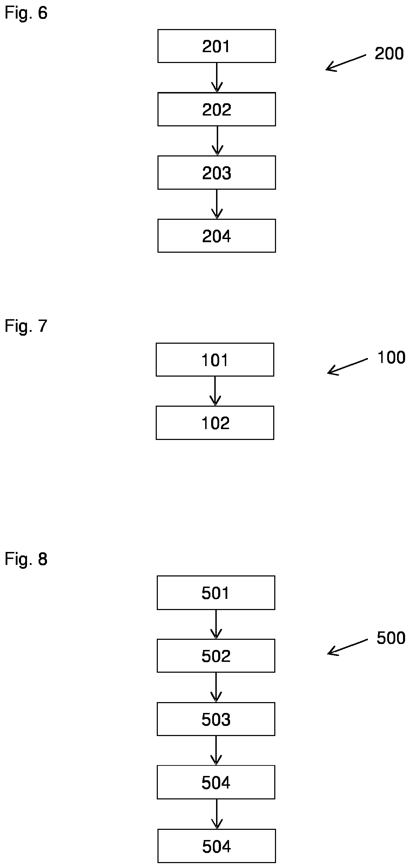

Fig. 7 zeigt ein Beispiel des erfindungsgemäßen Verfahrens 100 zum Einstellen einer Zieltemperatur $T_{ziel}$ in einer Kammer der erfindungsgemäßen Labortemperiervorrichtung, insbesondere einer erfindungsgemäßen Labortemperiervorrichtung gemäß Fig. 1 bis 5, aufweisend die Schritte

- Bestimmen eines vorrichtungsspezifischen Wertes $T_{x0}$, insbesondere mittels des Kalibrierverfahrens 200, wobei $T_{x0}$ so vorbestimmt wird, dass sich aus der Temperaturregelung $T_{ak} = T_{x0}$ die Einstellung der Temperatur $T_K$ im Kammerinnenraum auf $T_K = T_{ziel}$ ergibt. (101)
- Regelung der Außentemperatur $T_{ak}$ auf $T_{ak} = T_{x0}$ , wodurch sich die Einstellung der Temperatur $T_K$ im Kammerinnenraum auf $T_K = T_{ziel}$ ergibt. (102) Fig. 6 zeigt ein Beispiel des erfindungsgemäßen Kalibrierverfahrens 200 zum Kalibrieren einer erfindungsgemäßen Labortemperiervorrichtung gemäß Fig. 1 bis 5, aufweisend die Schritte
- Abwarten eines thermischen Gleichgewichts der Kammer; (201)
- verifiziertes Einstellen der Temperatur $T_K$ des Kammerinnenraums auf $T_K = T_{ziel}$ in einem thermischen Gleichgewicht der Kammer; (202)
- Bestimmung von $T_{x0}$ mittels eines äußeren Temperatursensors $T_{ak}$ an der Außenseite der Kammer der Labortemperiervorrichtung bei der nun zuverlässig eingestellten Kammerinnenraumtemperatur $T_K = T_{ziel}$. (203)
- Abspeichern von $T_{x0}$ in der Labortemperiervorrichtung bzw. deren Datenspeichereinrichtung. (204)

Das verifizierte Einstellen der Temperatur $T_K$ des Kammerinnenraums auf $T_K = T_{ziel}$ erfolgt hier, indem $T_K = T_{ziel}$ geregelt wird, indem ein zu Kalibrierzwecken in den Kammerinnenraum eingesetzter mobiler Temperatursensor $T_{innen}$ mit der elektrischen Steuereinrichtung der Labortemperiervorrichtung verbunden wird, so dass diese einen Regelkreis ausführt, bei dem der mobile Temperatursensor $T_{innen}$ im Kammerinnenraum als Messglied des Regelkreises arbeitet und die Temperiereinrichtung(en) als Stellglied des Regelkreises.

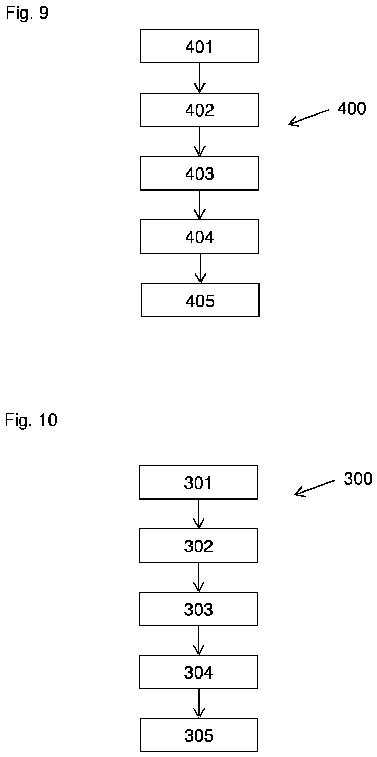

Fig. 10 zeigt ein Beispiel des erfindungsgemäßen Verfahrens 300 zur Schätzung der Temperatur $T_K$ im Innenraum der Kammer einer Labortemperiervorrichtung gemäß einem der Fig. 1 bis 5, aufweisend die Schritte:

- Bestimmen des vorrichtungsspezifischen Wertes $T_{x0}$, insbesondere gemäß dem in Anspruch 8 oder 9 beschriebenen Kalibrierverfahren, wobei $T_{x0}$ so vorbestimmt wird, dass sich aus der Temperaturregelung $T_{ak} = T_{x0}$ die Einstellung der Temperatur $T_K$ im Kammerinnenraum auf $T_K = T_{ziel}$ ergibt; (301)
- Bestimmen eines vorrichtungsspezifischen Wertes F; (302)
- insbesondere: Einstellung der Temperatur $T_K$ im Kammerinnenraum auf $T_K = T_{ziel}$ , durch die Temperaturregelung $T_{ak} = T_{x0}$ ; (303)

- insbesondere: Erfassen des Öffnens und Schließens der Kammertüre und/oder Gehäusetüre der Labortemperiervorrichtung; (304)
- Schätzung des Wertes $T_K$ im Innenraum der Kammer durch Berechnung, so dass

$$T_K = T_{Kb} = T_{Kb}(t) = T_{ak}(t) + T_{offset} - (P_{temp}(t) - P_{Basis}(t)) * F$$

wobei $T_{offset}$ definiert ist durch $T_{offset} = T_{x0} - T_{ziel}$, $P_{Basis}(t)$ ist die vor dem Öffnen der Kammertüre oder Gehäusetüre im stationären Zustand bei gegebener Umgebungstemperatur von der Temperiereinrichtung aufgebrachte Leistung. (305)

Fig. 9 zeigt ein Beispiel des erfindungsgemäßen Verfahrens 400 zur Ermittlung des vorrichtungsspezifischen Wertes F, mit den Schritten:

- Platzieren der Labortemperiervorrichtung in einer Umgebung mit einer bekannten Außentemperatur $T_{umgebung}$. (401)
- Verifiziertes Einstellen der Kammerinnenraumtemperatur $T_K$ auf einen Wert $T_K = T_{ziel\_0}$, wobei insbesondere $T_{umgebung} <> T_{ziel\_0}$ ; (402)
- Öffnen der Kammertüre und/oder einer Gehäusetüre zu einem bestimmten Zeitpunkt t0 und Schließen derselben zu einem späteren Zeitpunkt t1; (403)
- Vor und während dem Öffnen und Schließen und danach: Messen der Kammerinnenraumtemperatur $T_{innen}(t)$ und zeitabhängiges Speichern der Kammerinnenraumtemperatur $T_{innen}(t)$ ; (404)
- Verwenden einer Ermittlungsvorschrift, vorzugsweise einer Gleichung, insbesondere "Gleichung 1", für eine Ausgleichsrechnung zwischen der Gleichung und den Messwerten $T_{innen}(t)$, um den Faktor F zu ermitteln. (405)

Dabei erfolgt das verifizierte Einstellen der Kammerinnenraumtemperatur $T_K$ auf einen Wert $T_K = T_{ziel\_0}$ durch Einsetzen eines mobilen Temperatursensor $T_{innen}$ in den Kammerinnenraum und Verbinden des Temperatursensors $T_{innen}$ mit der elektrischen Steuereinrichtung der Labortemperiervorrichtung, so dass diese einen Regelkreis ausführt, bei dem der mobile Temperatursensor $T_{innen}$ im Kammerinnenraum als Messglied des Regelkreises arbeitet und die Temperiereinrichtung(en) als Stellglied des Regelkreises. Die Regelung erfolgt bei geschlossener Kammertüre und/oder Gehäusetüre.

Fig. 8 zeigt ein Beispiel eines erfindungsgemäßen Verfahrens (500) zur Durchführung einer Diagnose an einer erfindungsgemäßen Labortemperiervorrichtung zum Lagern von Laborproben bei einer Temperatur $T_{ziel}$, wobei die Labortemperiervorrichtung aufweist:

* eine Kammer zur Aufnahme von Laborproben im Kammerinnenraum, deren Außenseiten durch mindestens eine Kammerwand sowie eine Kammertüre gebildet werden, welche eine Kammeröffnung verschließt, durch die der Kammerinnenraum für den Benutzer zugänglich ist,
* eine Temperiereinrichtung zum Temperieren der Kammer, die im thermischen Kontakt zu einer Außenseite der Kammer angebracht ist,
* einen äußeren Temperatursensor, der im thermischen Kontakt zu einer Außenseite der Kammer zur Erfassung einer Temperatur $T_{ak}$ angebracht ist,
* einen inneren Temperatursensor, der im thermischen Kontakt mit dem Innenraum der Kammer zur Erfassung einer Temperatur $T_{innen}$ angebracht ist, der insbesondere im Innenraum platziert ist,
* eine Isolierungseinrichtung, welche die Temperiereinrichtung, den Temperatursensor und die Kammer gegenüber der Umgebung thermisch isoliert, und
* eine elektrische Steuereinrichtung, die dazu eingerichtet ist, die Temperatur $T_{ak}$ mittels des inneren Temperatursensors und der Temperiereinrichtung auf eine Solltemperatur mit dem Wert $T_{ziel}$ zu regeln, wobei in einer Datenspeichereinrichtung der vorrichtungsspezifische Wert $T_{x0}$ gespeichert ist,

wobei das Verfahren die Schritte aufweist:

- Regeln der Temperatur des Innenraums der Kammer auf den Wert $T_{ziel}$ ; (501)
- Messen der Temperatur $T_{ak}$ , wenn die Temperatur des Innenraums der Kammer auf den Wert $T_{ziel}$ geregelt ist und ein stationäres Gleichgewicht vorliegt, (502)
- Vergleichen von $T_{ak}$ mit $T_{x0}$, (503)
- Vorzugsweise: Speichern des Ergebnisses des Vergleichen von $T_{ak}$ mit $T_{x0}$, in einer Datenspeichereinrichtung der Labortemperiervorrichtung; (504)
- Vorzugsweise: Ausgabe einer Fehlermeldung mittels der Benutzerschnittstelleneinrichtung der Labortemperiervorrichtung, falls der Vergleich ergibt, dass $T_{ak} <> T_{x0}$. (505)

**Patentansprüche**

1.  Labortemperiervorrichtung (1) zum Temperieren von Laborproben bei einer Temperatur $T_{ziel}$, aufweisend

    * eine Kammer (2) zur Aufnahme von Laborproben im Kammerinnenraum (9), deren Außenseiten durch mindestens eine Kammerwand (2a) sowie eine Kammertüre (16b) gebildet werden, welche eine Kammeröffnung (2b) verschließt, durch die der Kammerinnenraum (9) für den Benutzer zugänglich ist,
    * eine Temperiereinrichtung (6) zum Temperieren der Kammer (2), die im thermischen Kontakt zu einer Außenseite der Kammer (2) angebracht ist,
    * einen Temperatursensor (3), der im thermischen Kontakt zu einer Außenseite der Kammer (2) zur Erfassung einer Temperatur $T_{ak}$ angebracht ist,
    * eine Isolierungseinrichtung (4), welche die Temperiereinrichtung (6), den Temperatursensor (3) und die Kammer (2) gegenüber der Umgebung thermisch isoliert, und
    * eine elektrische Steuereinrichtung (5), die dazu eingerichtet ist, die Temperatur $T_{ak}$ mittels des Temperatursensors (3) und der Temperiereinrichtung (6) auf eine Solltemperatur mit dem Wert $T_{x0}$ zu regeln, der so vorbestimmt ist, dass sich aus der Temperaturregelung $T_{ak} = T_{x0}$ die Einstellung der Temperatur $T_K$ im Kammerinnenraum (9) auf $T_K = T_{ziel}$ ergibt,
    wobei die Temperiereinrichtung (6) während der Temperaturregelung $T_{ak} = T_{x0}$ in Abhängigkeit von der Zeit t mit der elektrischen Leistung $P_{temp}(t)$ betrieben wird, und die elektrische Steuereinrichtung (5) diese Leistung $P_{temp}(t)$ und den zeitabhängigen Messwert $T_{ak}(t)$ erfasst und die elektrische Steuereinrichtung (5) eine Datenverarbeitungseinrichtung aufweist, die dazu eingerichtet ist, eine Temperatur $T_{Kb}$ als Temperatur des Kammerinnenraums (9) in Abhängigkeit von der Leistung $P_{temp}(t)$ und der gemessenen Temperatur $T_{ak}(t)$ zu berechnen.

2.  Labortemperiervorrichtung gemäß Anspruch 1, die keinen Temperatursensor aufweist, der die Temperatur im Kammerinnenraum (9) misst und als Messglied der Temperaturregelung der elektrischen Steuereinrichtung (5) dient, die $T_{ak}$ mittels des Temperatursensors (3) und der Temperiereinrichtung (6) auf eine Solltemperatur mit dem Wert $T_{x0}$ regelt.

3.  Labortemperiervorrichtung gemäß Anspruch 1 oder 2, dass die elektrische Steuereinrichtung (5) eine Datenspeichereinrichtung aufweist, in welcher der mindestens eine vorbestimmte Wert $T_{x0}$ gespeichert ist, der zur Temperaturregelung $T_{ak} = T_{x0}$ aus der Datenspeichereinrichtung entnommen wird.

4.  Labortemperiervorrichtung gemäß einem der vorangehenden Ansprüche,

    wobei die Labortemperiervorrichtung (1) einen Kammertürsensor aufweist, der das Öffnen und Schließen der Kammertüre erfasst
    oder
    die Labortemperiervorrichtung (1) ein Außengehäuse (7) mit einer Gehäusetüre (16), die im geöffneten Zustand dem Benutzer durch die geöffnete Kammertüre (16b) den Zugang zum Kammerinnenraum (9) ermöglicht, und einen Gehäusetürsensor aufweist, der das Öffnen und Schließen der Gehäusetüre (16) erfasst,
    wobei die elektrische Steuereinrichtung (5) dazu eingerichtet ist, das Öffnen und Schließen der Kammertüre (16b) oder der Gehäusetüre (16) zeitabhängig zu erfassen und die Temperatur $T_{Kb}$ als Temperatur des Kammerinnenraums (9) auch in Abhängigkeit von den Zeitpunkten des Öffnens und Schließens der Kammertüre (16b) oder der Gehäusetüre (16) zu berechnen.

5.  Labortemperiervorrichtung gemäß einem der vorangehenden Ansprüche, wobei die elektrische Steuereinrichtung (5) dazu eingerichtet ist, nach dem Öffnen und Schließen der Kammertüre (16b) oder einer Gehäusetüre (16) der Labortemperiervorrichtung (1) eine Temperatur $T_{Kb}(t)$ in Abhängigkeit von der Zeit t als Temperatur des Kammerinnenraums (9) gemäß

$$T_{Kb}(t) = T_{ak}(t) + T_{offset} - (P_{temp}(t) - P_{Basis}(t)) * F$$

zu berechnen, wobei $T_{offset}$ definiert ist durch $T_{offset} = T_{x0} - T_{ziel}$, $P_{Basis}(t)$ ist die vor dem Öffnen der Kammertüre (16b) oder Gehäusetüre (16) im stationären Zustand bei gegebener Umgebungstemperatur von der Temperiereinrichtung (6) aufgebrachte Leistung, und F ist ein Normierungsfaktor.

6. Verfahren (100) zum Einstellen einer Zieltemperatur $T_{ziel}$ in einer Kammer (2) einer Labortemperiervorrichtung (1) gemäß einem der Ansprüche 1 bis 5, aufweisend die Schritte:

• Bestimmen eines vorrichtungsspezifischen Wertes $T_{x0}$, wobei $T_{x0}$ so vorbestimmt wird, dass sich aus der Temperaturregelung $T_{ak} = T_{x0}$ die Einstellung der Temperatur $T_K$ im Kammerinnenraum (9) auf $T_K = T_{ziel}$ ergibt.
• Regelung der Außentemperatur $T_{ak}$ auf $T_{ak} = T_{x0}$, wodurch sich die Einstellung der Temperatur $T_K$ im Kammerinnenraum (9) auf $T_K = T_{ziel}$ ergibt,

wobei die Temperiereinrichtung (6) während der Temperaturregelung $T_{ak} = T_{x0}$ in Abhängigkeit von der Zeit t mit der elektrischen Leistung $P_{temp}(t)$ betrieben wird, und die elektrische Steuereinrichtung (5) diese Leistung $P_{temp}(t)$ und den zeitabhängigen Messwert $T_{ak}(t)$ erfasst und die elektrische Steuereinrichtung (5) eine Datenverarbeitungseinrichtung aufweist, die dazu eingerichtet ist, eine Temperatur $T_{Kb}$ als Temperatur des Kammerinnenraums (9) in Abhängigkeit von der Leistung $P_{temp}(t)$ und der gemessenen Temperatur $T_{ak}(t)$ zu berechnen.

7. Verfahren gemäß Anspruch 6, wobei $T_{x0}$ vorab in einem Kalibrierverfahren (200) bestimmt wird:

• Abwarten eines thermischen Gleichgewichts der Kammer (2);
• verifiziertes Einstellen der Temperatur $T_K$ des Kammerinnenraums (9) auf $T_K = T_{ziel}$ in einem thermischen Gleichgewicht der Kammer (2);
• Bestimmung von $T_{x0}$ mittels eines äußeren Temperatursensors (3) $T_{ak}$ an der Außenseite der Kammer (2) der Labortemperiervorrichtung (1) bei der nun zuverlässig eingestellten Kammerinnenraumtemperatur $T_K = T_{ziel}$.

8. Verfahren gemäß Anspruch 7, wobei $T_K = T_{ziel}$ geregelt wird, indem ein zu Kalibrierzwecken in den Kammerinnenraum (9) eingesetzter mobiler Temperatursensor $T_{in\text{-}nen}$ mit der elektrischen Steuereinrichtung (5) der Labortemperiervorrichtung (1) verbunden wird, so dass diese einen Regelkreis ausführt, bei dem der mobile Temperatursensor $T_{innen}$ im Kammerinnenraum als Messglied des Regelkreises arbeitet und die Temperiereinrichtung(en) (6) als Stellglied des Regelkreises.

9. Verfahren (300) zur Schätzung der Temperatur $T_K$ im Innenraum der Kammer (2) einer Labortemperiervorrichtung (1) gemäß einem der Ansprüche 1 bis 5, aufweisend die Schritte:

• Bestimmen des vorrichtungsspezifischen Wertes $T_{x0}$, insbesondere gemäß dem in Anspruch 8 oder 9 beschriebenen Kalibrierverfahren, wobei $T_{x0}$ so vorbestimmt wird, dass sich aus der Temperaturregelung $T_{ak} = T_{x0}$ die Einstellung der Temperatur $T_K$ im Kammerinnenraum (9) auf $T_K = T_{ziel}$ ergibt;
• Bestimmen eines vorrichtungsspezifischen Wertes F;
• insbesondere: Einstellung der Temperatur $T_K$ im Kammerinnenraum (9) auf $T_K = T_{ziel}$, durch die Temperaturregelung $T_{ak} = T_{x0}$ ;
• insbesondere: Öffnen und Schließen der Kammertüre (16b) und/oder Gehäusetüre (16) der Labortemperiervorrichtung (1);
• Schätzung des Wertes $T_K$ im Innenraum (9) der Kammer (2) durch Berechnung.

10. Verfahren gemäß Anspruch 9, wobei die Schätzung des Wertes $T_K$ im Innenraum (9) der Kammer (2) durch Berechnung erfolgt, so dass

$$T_K = T_{Kb} = T_{Kb}(t) = T_{ak}(t) + T_{offset} - (P_{temp}(t) - P_{Basis}(t)) * F \; ,$$

wobei $T_{offset}$ definiert ist durch $T_{offset} = T_{x0} - T_{ziel}$, $P_{Basis}(t)$ ist die vor dem Öffnen der Kammertüre (16b) oder Gehäusetüre (16) im stationären Zustand bei gegebener Umgebungstemperatur von der Temperiereinrichtung (6) aufgebrachte Leistung, und wobei F ein vorrichtungsspezifischer oder gerätespezifischer Wert ist.

11. Verfahren (400) gemäß Anspruch 10, wobei der Wert F bestimmt wird wie folgt:

• Platzieren der Labortemperiervorrichtung (1) in einer Umgebung mit einer bekannten Außentemperatur $T_{umgebung}$.
• Verifiziertes Einstellen der Kammerinnenraumtemperatur $T_K$ auf einen Wert $T_K = T_{ziel\_0}$, wobei insbesondere

$T_{umgebung} <> T_{ziel\_0}$;

• Öffnen der Kammertüre (16b) und/oder einer Gehäusetüre (16) zu einem bestimmten Zeitpunkt t0 und Schließen derselben zu einem späteren Zeitpunkt t1

• Vor und während dem Öffnen und Schließen und danach: Messen der Kammerinnenraumtemperatur $T_{innen}(t)$ und zeitabhängiges Speichern der Kammerinnenraumtemperatur $T_{innen}(t)$

• Verwenden einer Gleichung, insbesondere "Gleichung 1", für eine Ausgleichsrechnung zwischen der Gleichung und den Messwerten $T_{innen}(t)$, um den Faktor F zu ermitteln.

12. Verfahren gemäß Anspruch 11, wobei das verifizierte Einstellen der Kammerinnenraumtemperatur $T_K$ auf einen Wert $T_K = T_{ziel\_0}$ wie folgt durchgeführt wird:

• Einsetzen eines mobilen Temperatursensor $T_{innen}$ in den Kammerinnenraum (9) und Verbinden des Temperatursensors $T_{innen}$ mit der elektrischen Steuereinrichtung (5) der Labortemperiervorrichtung (1), so dass diese einen Regelkreis ausführt, bei dem der mobile Temperatursensor $T_{innen}$ im Kammerinnenraum (9) als Messglied des Regelkreises arbeitet und die Temperiereinrichtung(en) (6) als Stellglied des Regelkreises. Die Regelung erfolgt bei geschlossener Kammertüre (16b) und/oder Gehäusetüre (16).

13. Labortemperiervorrichtung gemäß einem der vorangehenden Ansprüche 1 bis 5, die ein Inkubator (1), insbesondere ein CO2-Inkubator für Zellkulturen ist.

14. Herstellen einer Labortemperiervorrichtung (1) gemäß einem der vorangehenden Ansprüche 1 bis 5, wobei der gerätespezifische Wert $T_{x0}$ vorab gemäß dem in Anspruch 7 oder 8 beschriebenen Kalibrierverfahren bestimmt wird.

15. Verfahren (500) zur Durchführung einer Diagnose an einer Labortemperiervorrichtung gemäß einem der Ansprüche 1 bis 5, wobei die Labortemperiervorrichtung zusätzlich einen inneren Temperatursensor aufweist, der im thermischen Kontakt mit dem Innenraum der Kammer zur Erfassung einer Temperatur $T_{innen}$ angebracht ist, der insbesondere im Innenraum platziert ist, wobei die elektrische Steuereinrichtung dazu eingerichtet ist, die Temperatur $T_{ak}$ mittels des inneren Temperatursensors und der Temperiereinrichtung auf eine Solltemperatur mit dem Wert $T_{ziel}$ zu regeln, wobei in einer Datenspeichereinrichtung der vorrichtungsspezifische Wert $T_{x0}$ gespeichert ist, wobei das Verfahren die Schritte aufweist:

• Regeln der Temperatur des Innenraums der Kammer auf den Wert $T_{ziel}$ ; (501)

• Messen der Temperatur $T_{ak}$, wenn die Temperatur des Innenraums der Kammer auf den Wert $T_{ziel}$ geregelt ist und ein stationäres Gleichgewicht vorliegt, (502)

• Vergleichen von $T_{ak}$ mit $T_{x0}$ . (503).

**Claims**

1. Laboratory temperature control device (1) for controlling the temperature of laboratory samples at a temperature $T_{Target}$, comprising

* a chamber (2) for receiving laboratory samples in the chamber interior (9), the outer sides of which are formed by at least one chamber wall (2a) and a chamber door (16b) which closes a chamber opening (2b) through which the chamber interior (9) is accessible to the user,
* a temperature control device (6) for controlling the temperature of the chamber (2), which is mounted in thermal contact with an outer side of the chamber (2),
* a temperature sensor (3), which is mounted in thermal contact with an outer side of the chamber (2) to detect a temperature $T_{ak}$,
* an insulating device (4) which thermally insulates the temperature control device (6), the temperature sensor (3) and the chamber (2) from the environment, and
* an electrical control device (5), which is set up to regulate the temperature $T_{ak}$ by means of the temperature sensor (3) and the temperature control device (6) to a setpoint temperature with the value $T_{x0}$, which is predetermined in such a way that the setting of the temperature $T_K$ in the chamber interior (9) to $T_{x0} = T_{Target}$ results from the temperature regulation $T_K = T_{Target}$,

wherein the temperature control device (6) is operated with the electrical power $P_{temp}(t)$ during the temperature control $T_{ak} = T_{x0}$ as a function of the time t, and the electrical control device (5) detects this power $P_{temp}(t)$ and the time-dependent measured value $T_{ak}(t)$ and the electrical control device (5) comprises a data processing device which is set up to calculate a temperature $T_{Kb}$ as the temperature of the chamber interior (9) as a function of the power $P_{temp}$ (t) and the measured temperature $T_{ak}$ (t).

2. Laboratory temperature control device according to claim 1, which comprises no temperature sensor which measures the temperature in the chamber interior (9) and serves as a measuring element of the temperature control of the electrical control device (5), which regulates $T_{ak}$ to a setpoint temperature with the value $T_{x0}$ by means of the temperature sensor (3) and the temperature control device (6).

3. Laboratory temperature control device according to claim 1 or 2, in that the electrical control device (5) comprises a data storage device in which the at least one predetermined value $T_{x0}$ is stored, which is taken from the data storage device for temperature control $T_{ak} = T_{x0}$.

4. Laboratory temperature control device according to any of the preceding claims,

   wherein the laboratory temperature control device (1) comprises a chamber door sensor which detects the opening and closing of the chamber door
   or
   the laboratory temperature control device (1) comprises an outer housing (7) with a housing door (16) which, when open, allows the user access to the chamber interior (9) through the open chamber door (16b), and a housing door sensor which detects the opening and closing of the housing door (16),
   wherein the electrical control device (5) is set up to detect the opening and closing of the chamber door (16b) or the housing door (16) as a function of time and to calculate the temperature $T_{Kb}$ as the temperature of the chamber interior (9) also as a function of the times of opening and closing of the chamber door (16b) or the housing door (16).

5. Laboratory temperature control device according to one of the preceding claims, wherein the electrical control device (5) is set up, after opening and closing the chamber door (16b) or a housing door (16) of the laboratory temperature control device (1), to set a temperature $T_{Kb}$ (t) as a function of the time t as the temperature of the chamber interior (9) according to claim 1.

$$T_{Kb} (t) = T_{ak} (t) + T_{offset} - (P_{temp} (t) - P_{Basis} (t)) * F$$

   wherein $T_{offset}$ is defined by $T_{offset} = T_{x0} - T_{Target}$, $P_{Basis}$ (t) is the power applied by the temperature control device (6) before the chamber door (16b) or housing door (16) is opened in the stationary state at a given ambient temperature, and F is a normalization factor.

6. Method (100) for setting a target temperature $T_{Target}$ in a chamber (2) of a laboratory temperature control device (1) according to any one of claims 1 to 5, comprising the steps of:

   • Determining a device-specific value $T_{x0}$, wherein $T_{x0}$ is predetermined in such a way that the temperature control $T_{ak} = T_{x0}$ results in the temperature $T_K$ in the chamber interior (9) being set to $T_K = T_{Target}$.
   • Regulating of the outside temperature $T_{ak}$ to $T_{ak} = T_{x0}$, resulting in the setting of the temperature $T_{ak}$ in the chamber interior (9) to $T_K = T_{Target}$,
   wherein the temperature control device (6) is operated with the electrical power $P_{temp}$ (t) during the temperature control $T_{ak} = T_{x0}$ as a function of the time t, and the electrical control device (5) detects this power $P_{temp}$ (t) and the time-dependent measured value $T_{ak}$(t) and the electrical control device (5) comprises a data processing device which is set up to calculate a temperature $T_{Kb}$ as the temperature of the chamber interior (9) as a function of the power $P_{temp}$ (t) and the measured temperature $T_{ak}$(t).

7. Method according to claim 6, wherein $T_{x0}$ is determined in advance in a calibration method (200):

   • Waiting for the chamber (2) to reach thermal equilibrium;
   • verified setting of the temperature $T_K$ of the chamber interior (9) to $T_K = T_{Target}$ in a thermal equilibrium of the chamber (2);
   • Determining of $T_{x0}$ by means of an external temperature sensor (3) $T_{ak}$ on the outside of the chamber (2) of the laboratory temperature control device (1) at the now reliably set chamber interior temperature $T_K = T_{Target}$.

8. Method according to claim 7, wherein $T_K = T_{Target}$ is controlled by connecting a mobile temperature sensor $T_{inside}$ inserted into the chamber interior (9) for calibration purposes to the electrical control device (5) of the laboratory temperature control device (1), so that the latter executes a control loop in which the mobile temperature sensor $T_{inside}$ in the chamber interior operates as a measuring element of the control loop and the temperature control device(s) (6) operates as an actuator of the control loop.

9. Method (300) for estimating the temperature $T_K$ in the interior of the chamber (2) of a laboratory temperature control device (1) according to any one of claims 1 to 5, comprising the steps of:

  • Determining the device-specific value $T_{x0}$, in particular according to the calibration method described in claim 8 or 9, wherein $T_{x0}$ is predetermined such that the setting of the temperature $T_{x0}$ in the chamber interior (9) to $T_{ak} = T_{x0}$ results from the temperature control $T_K = T_{Target}$,
  • Determining a device-specific value F;
  • in particular: Setting the temperature $T_K$ in the chamber interior (9) to $T_K = T_{Target}$, by the temperature control $T_{ak} = T_{x0}$;
  • in particular: Opening and closing the chamber door (16b) and/or housing door (16) of the laboratory temperature control device (1);
  • Estimating the value $T_K$ in the interior (9) of the chamber (2) by calculation.

10. Method according to claim 9, wherein the estimation of the value $T_K$ in the interior (9) of the chamber (2) is carried out by calculation, so that

$$T_K = T_{Kb} = T_{Kb}(t) = T_{ak}(t) + T_{offset} - (P_{temp}(t) - P_{Basis}(t)) * F,$$

wherein $T_{offset}$ is defined by $T_{offset} = T_{x0} - T_{Target}$, $P_{Basis}(t)$ is the power applied by the temperature control device (6) before the chamber door (16b) or housing door (16) is opened in the stationary state at a given ambient temperature, and where F is a device-specific or appliance-specific value.

11. Method (400) according to claim 10, wherein the value F is determined as follows:

  • Placing the laboratory temperature control unit (1) in an environment with a known outside temperature $T_{environment}$.
  • Verified setting of the chamber interior temperature $T_K$ to a value $T_K = T_{Target\_0}$, where in particular $T_{environment} <> T_{Target\_0}$ ;
  • Opening the chamber door (16b) and/or a housing door (16) at a specific time t0 and closing it at a later time t1
  • Before and during opening and closing and afterwards: Measuring of the chamber interior temperature $T_{inside}(t)$ and time-dependent storage of the chamber interior temperature $T_{inside}(t)$
  • Use an equation, in particular "Equation 1", for a balancing calculation between the equation and the measured values $T_{inside}(t)$ to determine the factor F.

12. The method according to claim 11, wherein the verified setting of the chamber interior temperature $T_K$ to a value $T_K = T_{target\_0}$ is carried out as follows:

  • Inserting a mobile temperature sensor $T_{inside}$ into the chamber interior (9) and connecting the temperature sensor $T_{inside}$ to the electrical control device (5) of the laboratory temperature control device (1) so that the latter executes a control loop in which the mobile temperature sensor $T_{inside}$ in the chamber interior (9) acts as a measuring element of the control loop and the temperature control device(s) (6) acts as an actuator of the control loop. Control takes place when the chamber door (16b) and/or housing door (16) is closed.

13. Laboratory temperature control device according to any one of the preceding claims 1 to 5, which is an incubator (1), in particular a CO2 incubator for cell cultures.

14. Manufacturing of a laboratory temperature control device (1) according to any one of the preceding claims 1 to 5, wherein the device-specific value $T_{x0}$ is determined in advance according to the calibration method described in claim 7 or 8.

15. Method (500) for carrying out a diagnosis on a laboratory temperature control device according to one of claims 1 to 5, wherein the laboratory temperature control device additionally comprises an internal temperature sensor which is mounted in thermal contact with the interior of the chamber for detecting a temperature $T_{inside}$, which is placed in particular in the interior, wherein the electrical control device is set up to regulate the temperature $T_{ak}$ by means of the internal temperature sensor and the temperature control device to a setpoint temperature with the value $T_{Target}$, wherein the device-specific value $T_{x0}$ is stored in a data storage device,
wherein the method comprises the steps of:

- Controlling the temperature of the interior of the chamber to the value $T_{Target}$, (501)
- Measuring of the temperature $T_{ak}$, if the temperature of the chamber interior is controlled to the value $T_{Target}$ and a stationary equilibrium exists, (502)
- Comparing of $T_{ak}$ with $T_{Target}$. (503).

## Revendications

1. Dispositif de mise en température de laboratoire (1) pour mettre en température des échantillons de laboratoire à une température $T_{ziel}$, comprenant

   • une chambre (2) pour la réception d'échantillons de laboratoire dans l'espace interne de la chambre (9), dont les faces externes sont formées par au moins une paroi de chambre (2a) et une porte de chambre (16b), qui ferme une ouverture de chambre (2b), par laquelle l'espace interne de la chambre (9) est accessible à l'utilisateur,
   • un système de mise en température (6) pour mettre en température la chambre (2), qui est mis en contact thermique avec une face externe de la chambre (2),
   • un capteur de température (3) qui est mis en contact thermique avec une face externe de la chambre (2) pour la détection d'une température $T_{ak}$,
   • un système d'isolation (4) qui isole thermiquement vis-à-vis de l'environnement le système de mise en température (6), le capteur de température (3) et la chambre (2), et
   • un système de commande électrique (5) qui est conçu pour régler la température $T_{ak}$ au moyen du capteur de température (3) et du système de mise en température (6) sur une température de consigne avec la valeur $T_{x0}$ qui est prédéfinie pour qu'il résulte du réglage de la température $T_{ak} = T_{x0}$ l'ajustement de la température $T_K$ dans l'espace interne de la chambre (9) sur $T_K = T_{ziel}$,

   dans lequel le système de mise en température (6) fonctionne pendant le réglage de température $T_{ak} = T_{x0}$ en fonction du temps t avec la puissance électrique $P_{temp}(t)$, et le système de commande électrique (5) détecte cette puissance $P_{temp}(t)$ et la valeur de mesure en fonction du temps $T_{ak}(t)$ et le système de commande électrique (5) comprend un système de traitement de données qui est conçu pour calculer une température $T_{Kb}$ en tant que température de l'espace interne de la chambre (9) en fonction de la puissance $P_{temp}(t)$ et de la température mesurée $T_{ak}(t)$ .

2. Dispositif de mise en température de laboratoire selon la revendication 1, qui ne comprend pas de capteur de température, qui mesure la température dans l'espace interne de la chambre (9) et sert d'élément de mesure du réglage de température du système de commande électrique (5), qui règle $T_{ak}$ au moyen du capteur de température (3) et du système de mise en température (6) sur une température de consigne avec la valeur $T_{x0}$.

3. Dispositif de mise en température de laboratoire selon la revendication 1 ou 2, dans lequel le système de commande électrique (5) comprend un système d'enregistrement de données dans lequel la au moins une valeur prédéfinie $T_{x0}$ est enregistrée, qui est extraite du système d'enregistrement de données pour le réglage de la température $T_{ak} = T_{x0}$.

4. Dispositif de mise en température de laboratoire selon l'une des revendications précédentes,

   ledit dispositif de mise en température (1) comprenant un capteur de porte de chambre qui détecte l'ouverture et la fermeture de la porte de chambre
   ou
   ledit dispositif de mise en température (1) comprenant un boîtier externe (7) avec une porte de boîtier (16), qui, à l'état ouvert, permet à l'utilisateur d'accéder à l'espace interne de la chambre (9) à travers la porte de chambre ouverte (16b), et comprenant un capteur de porte de boîtier qui détecte l'ouverture et la fermeture de la porte de boîtier (16),

dans lequel le système de commande électrique (5) est conçu pour détecter en fonction du temps l'ouverture et la fermeture de la porte de chambre (16b) ou de la porte de boîtier (16) et calculer la température $T_{Kb}$ en tant que température de l'espace interne de la chambre (9) également en fonction des moments d'ouverture et de fermeture de la porte de chambre (16b) ou de la porte de boîtier (16).

**5.** Dispositif de mise en température de laboratoire selon l'une des revendications précédentes, dans lequel ledit système de commande électrique (5) est conçu pour calculer, après l'ouverture et la fermeture de la porte de chambre (16b) ou d'une porte de boîtier (16) du dispositif de mise en température de laboratoire (1), une température $T_{Kb}(t)$ en fonction du temps t en tant que température de l'espace interne de la chambre (9) d'après

$$T_{Kb}(t) = T_{ak}(t) + T_{offset} - \left(P_{temp}(t) - P_{Basis}(t)\right) * F$$

où $T_{offset}$ est définie par $T_{offset} = T_{x0} - T_{ziel}$, $P_{Basis}(t)$ est la puissance fournie par le système de mise en température (6) avant l'ouverture de la porte de chambre (16b) ou de la porte de boîtier (16) à l'état stationnaire pour une température d'environnement donnée, et F est un facteur de normalisation.

**6.** Procédé (100) de réglage d'une température cible $T_{ziel}$ dans une chambre (2) d'un dispositif de mise en température de laboratoire (1) selon l'une des revendications 1 à 5, comprenant les étapes consistant à :

• déterminer une valeur spécifique à un dispositif $T_{x0}$, où $T_{x0}$ est prédéfinie de manière à ce qu'il résulte du réglage de la température $T_{ak} = T_{x0}$ l'ajustement de la température $T_K$ dans l'espace interne de la chambre (9) sur $T_K = T_{ziel}$.
• régler la température externe $T_{ak}$ sur $T_{ak} = T_{x0}$, moyennant quoi il en résulte le réglage de la température $T_K$ dans l'espace interne de la chambre (9) sur $T_K = T_{ziel}$,

dans lequel le système de mise en température (6) fonctionne pendant le réglage de température $T_{ak} = T_{x0}$ en fonction du temps t avec la puissance électrique $P_{temp}(t)$, et le système de commande électrique (5) détecte cette puissance $P_{temp}(t)$ et la valeur de mesure en fonction du temps $T_{ak}(t)$ et le système de commande électrique (5) comprend un système de traitement de données qui est conçu pour calculer une température $T_{Kb}$ en tant que température de l'espace interne de la chambre (9) en fonction de la puissance $P_{temp}(t)$ et de la température mesurée $T_{ak}(t)$ .

**7.** Procédé selon la revendication 6, dans lequel $T_{x0}$ est définie au préalable dans une procédure de calibrage (200) dont les étapes sont :

• attente d'un équilibre thermique de la chambre (2) ;
• ajustement vérifié de la température $T_K$ de l'espace interne de la chambre (9) sur $T_K = T_{ziel}$ dans un équilibre thermique de la chambre (2) ;
• détermination de $T_{x0}$ au moyen d'un capteur de température externe (3) $T_{ak}$ sur la face externe de la chambre (2) du dispositif de mise en température de laboratoire (1) pour la température de l'espace interne de la chambre à présent ajustée de manière fiable $T_K = T_{ziel}$.

**8.** Procédé selon la revendication 7, dans lequel $T_K = T_{ziel}$ est réglée en reliant un capteur mobile de température $T_{innen}$ mis en œuvre à des fins de calibrage dans l'espace interne de la chambre (9) avec le système de commande électrique (5) du dispositif de mise en température de laboratoire (1) de telle sorte que celui-ci exécute une boucle d'asservissement dans laquelle le capteur mobile de température $T_{innen}$ travaille dans l'espace interne de la chambre comme élément de mesure de la boucle d'asservissement et le ou les systèmes de mise en température (6) comme élément(s) de réglage de la boucle d'asservissement.

**9.** Procédé (300) d'évaluation de la température $T_K$ dans l'espace interne de la chambre (2) d'un dispositif de mise en température de laboratoire (1) selon l'une des revendications 1 à 5, comprenant les étapes de :

• détermination de la valeur spécifique au dispositif $T_{x0}$, en particulier selon le procédé de calibrage décrit dans la revendication 8 ou 9, où $T_{x0}$ est prédéfinie de sorte qu'il résulte du réglage de la température $T_{ak} = T_{x0}$ l'ajustement de la température $T_K$ dans l'espace interne de la chambre (9) sur $T_K = T_{ziel}$ ;
• détermination d'une valeur spécifique au dispositif F ;

• en particulier : ajustement de la température $T_K$ dans l'espace interne de la chambre (9) sur $T_K = T_{ziel}$, par réglage de la température $T_{ak} = T_{x0}$ ;
• en particulier : ouverture et fermeture de la porte de chambre (16b) et/ou de la porte de boîtier (16) du dispositif de mise en température de laboratoire (1) ;
• évaluation de la valeur $T_K$ dans l'espace interne (9) de la chambre (2) par calcul.

10. Procédé selon la revendication 9, dans lequel l'évaluation de la valeur $T_K$ dans l'espace interne (9) de la chambre (2) s'effectue par calcul de telle sorte que :

$$T_K = T_{Kb} = T_{Kb}(t) = T_{ak}(t) + T_{offset} - \left( P_{temp}(t) - P_{Basis}(t) \right) * F ,$$

où $T_{offset}$ est définie par $T_{offset} = T_{x0} - T_{ziel}$, $P_{Basis}(t)$ est la puissance fournie par le système de mise en température (6) avant l'ouverture de la porte de chambre (16b) ou de la porte de boîtier (16) à l'état stationnaire pour une température d'environnement donnée, et où F est une valeur spécifique au dispositif ou spécifique à l'appareil.

11. Procédé (400) selon la revendication 10, dans lequel la valeur F est déterminée par les étapes suivantes :

• placement du dispositif de mise en température de laboratoire (1) dans un environnement ayant une température externe connue $T_{umgebung}$ ;
• ajustement vérifié de la température de l'espace interne de la chambre $T_K$ sur une valeur $T_K = T_{ziel\,0}$, où en particulier $T_{umgebung} <> T_{ziel\,0}$ ;
• ouverture de la porte de chambre (16b) et/ou de la porte de boîtier (16) à un moment déterminé t0 et fermeture de celles-ci à un moment ultérieur t1
• avant et pendant l'ouverture et la fermeture et après : mesure de la température de l'espace interne de la chambre $T_{innen}(t)$ et enregistrement en fonction du temps de la température de l'espace interne de la chambre $T_{innen}(t)$
• utilisation d'une égalité, en particulier d'une « égalité 1 », pour un calcul de compensation entre l'égalité et les valeurs de mesures $T_{innen}(t)$ pour déterminer le facteur F.

12. Procédé selon la revendication 11, dans lequel l'ajustement vérifié de la température de l'espace interne de la chambre $T_K$ sur une valeur $T_K = T_{ziel\,0}$ est réalisé par les étapes suivantes :

• mise en œuvre d'un capteur mobile de température $T_{innen}$ dans l'espace interne de la chambre (9) et liaison du capteur de température $T_{innen}$ avec le système de commande électrique (5) du dispositif de mise en température de laboratoire (1) de telle sorte que celui-ci exécute une boucle d'asservissement, dans laquelle le capteur mobile de température $T_{innen}$ travaille dans l'espace interne de la chambre (9) en tant qu'élément de mesure de la boucle d'asservissement et le ou les systèmes de mise en température (6) comme élément(s) de réglage de la boucle d'asservissement. Le réglage s'effectue lorsque la porte de chambre (16b) et/ou la porte de boîtier (16) est fermée.

13. Dispositif de mise en température de laboratoire selon l'une des revendications précédentes 1 à 5, qui est un incubateur (1), en particulier un incubateur au $CO_2$ pour des cultures cellulaires.

14. Fabrication d'un dispositif de mise en température de laboratoire (1) selon l'une des revendications précédentes 1 à 5, dans laquelle la valeur spécifique à l'appareil $T_{x0}$ est déterminée au préalable selon le procédé de calibrage décrit dans la revendication 7 ou 8.

15. Procédé (500) de réalisation d'un diagnostic sur un dispositif de mise en température de laboratoire selon l'une des revendications 1 à 5, dans lequel le dispositif de mise en température de laboratoire comprend en plus un capteur de température interne qui est mis en contact thermique avec l'espace interne de la chambre afin de détecter une température $T_{innen}$, qui est placé en particulier dans l'espace interne, dans lequel le système de commande électrique est conçu pour régler la température $T_{ak}$ au moyen du capteur de température interne et du système de mise en température sur une température de consigne avec la valeur $T_{ziel}$, où la valeur spécifique au dispositif $T_{x0}$ est enregistrée dans un système d'enregistrement de données,
le procédé comprenant les étapes de :

• réglage de la température de l'espace interne de la chambre sur la valeur $T_{ziel}$ ; (501)
• mesure de la température $T_{ak}$, quand la température de l'espace interne de la chambre est réglée sur la valeur $T_{ziel}$ et qu'il existe un équilibre stationnaire, (502)
• comparaison de $T_{ak}$ avec $T_{x0}$ (503) .

Fig. 1a

Fig. 1b

Fig. 2

Fig. 3

Fig. 4

Fig. 5

Fig. 6

```
┌──────────┐
│   201    │
└──────────┘
     │
     ▼
┌──────────┐
│   202    │        ← 200
└──────────┘
     │
     ▼
┌──────────┐
│   203    │
└──────────┘
     │
     ▼
┌──────────┐
│   204    │
└──────────┘
```

Fig. 7

```
┌──────────┐
│   101    │
└──────────┘        ← 100
     │
     ▼
┌──────────┐
│   102    │
└──────────┘
```

Fig. 8

```
┌──────────┐
│   501    │
└──────────┘
     │
     ▼
┌──────────┐
│   502    │        ← 500
└──────────┘
     │
     ▼
┌──────────┐
│   503    │
└──────────┘
     │
     ▼
┌──────────┐
│   504    │
└──────────┘
     │
     ▼
┌──────────┐
│   504    │
└──────────┘
```

Fig. 9

```
┌─────────┐
│   401   │
└─────────┘
     │
     ▼
┌─────────┐
│   402   │        ←  400
└─────────┘
     │
     ▼
┌─────────┐
│   403   │
└─────────┘
     │
     ▼
┌─────────┐
│   404   │
└─────────┘
     │
     ▼
┌─────────┐
│   405   │
└─────────┘
```

Fig. 10

```
┌─────────┐
│   301   │        ←  300
└─────────┘
     │
     ▼
┌─────────┐
│   302   │
└─────────┘
     │
     ▼
┌─────────┐
│   303   │
└─────────┘
     │
     ▼
┌─────────┐
│   304   │
└─────────┘
     │
     ▼
┌─────────┐
│   305   │
└─────────┘
```

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6518059 B1
- US 6063619 A
- GB 2438683 A